# EUROPEAN PATENT SPECIFICATION

(11) **EP 0 824 541 B1**
(45) Date of publication and mention of the grant of the patent: **16.12.2009**
(21) Application number: 96915463.2
(22) Date of filing: 02.05.1996
(51) Int. Cl.: C07H 21/02, C07H 21/04, C12P 19/34, C12Q 1/68

(54) **NUCLEIC ACID LIGAND COMPLEXES**
NUKLEINSÄURELIGAND-KOMPLEXE
COMPLEXES DE LIGANDS D'ACIDE NUCLEIQUE

(30) Priority: 04.05.1995 US 434465; 05.06.1995 US 464443
(43) Date of publication of application: 25.02.1998
(73) Proprietor: GILEAD SCIENCES, INC., Foster City CA 94404 (US)
(72) Inventor: GOLD, Larry, Boulder, CO 80302 (US); SCHMIDT, Paul, G., San Marino, CA 91108 (US); JANJIC, Nebojsa, Boulder, CO 80301 (US)
(74) Representative: Clegg, Richard Ian
(86) International application number: PCT/US1996/006171
(87) International publication number: WO 1996/034876

(56) References cited:
- WO-A-92/14843
- WO-A-92/14843
- WO-A-94/08050
- US-A- 5 270 163
- US-A- 5 270 163
- JÄSCHKE A. ET AL: "Synthesis and properties of oligodeoxyribonucleotides polyethylene glycol conjugates" NUCLEIC ACID RESEARCH, vol. 22, no. 22, 1994, - 4810 page 4817, XP008098272
- SUSAN WANG ET AL: "Delivery of antisense oligodeoxyribonulceotides against the human epidermal growth factor receptor into cultured KB cells with liposomes conjugated to folate via PEG" BIOCHEMISTRY, vol. 92, 1995, pages 3318-3322, XP002098092
- CHEMICAL ABSTRACTS, vol. 121, no. 83819, 1993, Columbus, Ohio, US; abstract no.: 121:83819,

### SEQUENCE LISTING

(1) GENERAL INFORMATION:
   (i) APPLICANT: LARRY GOLD, PAUL G. SCHMIDT, NEBOJSA JANJIC
   (ii) TITLE OF INVENTION: NUCLEIC ACID LIGAND COMPLEXES
   (iii) NUMBER OF SEQUENCES: 30
   (iv) CORRESPONDENCE ADDRESS:
      (A) ADDRESSEE: Swanson and Bratschun, L.L.C.
      (B) STREET: 8400 East Prentice Avenue, Suite #200
      (C) CITY: Denver
      (D) STATE: Colorado
      (E) COUNTRY: USA
      (F) ZIP: 80111
   (v) COMPUTER READABLE FORM:
      (A) MEDIUM TYPE: Diskette, 3.50 inch, 1.44 Mb storage
      (B) COMPUTER: IBM compatible
      (C) OPERATING SYSTEM: MS-DOS
      (D) SOFTWARE: WordPerfect 8.0
   (vi) CURRENT APPLICATION DATA:
      (A) APPLICATION NUMBER: 08/945,604
      (B) FILING DATE: 28-OCTOBER-1997
      (C) CLASSIFICATION:
   (vii)PRIOR APPLICATION DATA:
      (A) APPLICATION NUMBER: 08/434,465
      (B) FILING DATE: 04-MAY-1995
   (vii)PRIOR APPLICATION DATA:
      (A) APPLICATION NUMBER: 08/464,443
      (B) FILING DATE: 05-JUNE-1995
   (viii)ATTORNEY/AGENT INFORMATION:
      (A) NAME: BARRY J. SWANSON
      (B) REGISTRATION NUMBER: 33,215
      (C) REFERENCE/DOCKET NUMBER: NEX29C/US
   (ix) TELECOMMUNICATION INFORMATION:
      (A) TELEPHONE: (303) 793-3333
      (B) TELEFAX: (303) 793-3433
(2) INFORMATION FOR SEQ ID NO:1:
   (i) SEQUENCE CHARACTERIZATION:
      (A) LENGTH: 23
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:1:
      TTTTTTTTGT CAGGCACCAT CCC 23
(2) INFORMATION FOR SEQ ID NO:2:
   (i) SEQUENCE CHARACTERIZATION:
      (A) LENGTH: 25
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:2:
      CCCCACTGAA GCACCTTTTT TTTTT 25
(2) INFORMATION FOR SEQ ID NO:3:
   (i) SEQUENCE CHARACTERIZATION:
      (A) LENGTH: 36
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:3:
      CAGTCCGTGG TAGGGCAGGT TGGGGTGACT TCGTGG 36
(2) INFORMATION FOR SEQ ID NO:4:
   (i) SEQUENCE CHARACTERIZATION:
      (A) LENGTH: 53
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides 49-53 are bound by a phosphorothioate linkage
      (xi) SEQUENCE DESCRIPTION: SEQ ID NO:4:
   ATCCGCCTGA TTAGCGATAC TCAGAAGGAT AAACTGTCCA GAACTTGGAT 50
   TTT 53
(2) INFORMATION FOR SEQ ID NO:5:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide 39 is an inverted-orientation (3'3'linkage) phosphoramidite
   (xi) SEQUENCE DESCRIPTION: SEQ ID NO:5:
      CAGTCCGTGG TAGGGCAGGT TGGGGTGACT TCGTGGAATT 40
(2) INFORMATION FOR SEQ ID NO:6:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
   (D) OTHER INFORMATION: Nucleocides 37 and 38 are bound by a phosphorothioate bond
   (xi) SEQUENCE DESCRIPTION: SEQ ID NO:6:
      CAGTCCGTGG TAGGGCAGGT TGGGGTGACT TCGTGGAA 38
(2) INFORMATION FOR SEQ ID NO:7:
   (i) SEQUENCE CHARACTERIZATION:
      (A) LENGTH: 39
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:7:
      CAGTCCGTGG TAGGGCAGGT TGGGGTGACT TCGTGGAAT 39
(2) INFORMATION FOR SEQ ID NO:8:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 39 is an inverted-orientation (3'3' linkage) phosphoramidite
      (xi) SEQUENCE DESCRIPTION: SEQ ID NO:8:
         CAGTCCGTGG TAGGGCAGGT TGGGGTGACT TCGTGGAATT 40
(2) INFORMATION FOR SEQ ID NO:9:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
   (ix) FEATURE:
      (D) OTHER INFORMATION: Nucleotide number 37 is an inverted-orientation (3'3' linkage) phosphoramidite
   (xi) SEQUENCE DESCRIPTION: SEQ ID NO:9:
      CAGTCCGTGG TAGGGCAGGT TGGGGTGACT TCGTGGTT 38
(2) INFORMATION FOR SEQ ID NO:10:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:10:
      GCGGGGCTAC GTACCGGGGC TTTGTAAAAC CCCGC 35
(2) INFORMATION FOR SEQ ID NO:11:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:11:
      GCGGGGCTAC GTACCGGGGC TTTGTAAAAC CCCGC 35
(2) INFORMATION FOR SEQ ID NO:12:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 39 is an inverted orientation phosphoramidite
   (xi) SEQUENCE DESCRIPTION: SEQ ID NO:12:
      TCAGTCCGTG GTAGGGCAGG TTGGGGTGAC TTCGTGGATT 40
(2) INFORMATION FOR SEQ ID NO:13:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide 37 is an inverted orientation (3'3' linkage) phosphoramidite

      (xi) SEQUENCE DESCRIPTION: SEQ ID NO:13:
      CAGTCCGTGG TAGGGCAGGT TGGGGTGACT TCGTGGTT 38
(2) INFORMATION FOR SEQ ID NO:14:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:14:
      GCGGGGCTAC GTACCGGGGC TTTGTAAAAC CCCGC 35
(2) INFORMATION FOR SEQ ID NO:15:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 37 is an inverted orientation (3'3' linkage) phosphoramidite
   (xi) SEQUENCE DESCRIPTION: SEQ ID NO:15:
      TCAGTCCGTG GTAGGGCAGG TTGGGGTGAC TTCGTGTT 38
(2) INFORMATION FOR SEQ ID NO:16:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2-OMethyl (2'-OMe) modified.
   (ix) FEATURE:
      (D) OTHER INFORMATION: Nucleotides at positions 6-9, 12, 15, 19, 21, 22, and 27 are 2'-amino (2'-NH₂) modified.
   (xi) SEQUENCE DESCRIPTION: SEQ ID NO:16:
      TTTTACCCUG AUGGUAGACG CCGGGGUGTT TTT 33
(2) INFORMATION FOR SEQ ID NO:17:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5-28 are 2'-OMethyl (2'-OMe) modified.
   (xi) SEQUENCE DESCRIPTION: SEQ ID NO:17:
      TTTTACCCUG AUGGUAGACG CCGGGGUGTT TTT 33
(2) INFORMATION FOR SEQ ID NO:18:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:18:
      CAGTCCGTGG TAGGGCAGGT TGGGGTGACT TCGTGGAA 38
(2) INFORMATION FOR SEQ ID NO:19:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2'-OMethyl(2'-OMe) modified.
   (ix) FEATURE:
      (D) OTHER INFORMATION: Nucleotides at positions 6-9, 12, 15, 19, 21, 22 and 27 are 2'-amino (2'-NH₂) modified.
   (xi) SEQUENCE DESCRIPTION: SEQ ID NO:19:
      TTTTACCCUG AUGGUAGACG CCGGGGUGTT TTT 33
(2) INFORMATION FOR SEQ ID NO:20:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: All nucleotides are 2'-OMethyl (2'-OMe) modified.
   (xi) SEQUENCE DESCRIPTION: SEQ ID NO:20:
      UUUUACCCUG AUGGUAGACG CCGGGGUG 28
(2) INFORMATION FOR SEQ ID NO: 21:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond
   (ix) FEATURE:
      (D) OTHER INFORMATION: Nucleotides at positions 5, 13, 16, 17, 20, 23-26, and 28 are 2'-OMethyl (2'-OMe) modified
   (ix) FEATURE:
      (D) OTHER INFORMATION: Nucleotides at positions 6-9, 12, 15, 19, 21, 22 and 27 are 2'-amino (2'-NH₂) modified
   (xi) SEQUENCE DESCRIPTION: SEQ ID NO: 21:
      TTTTACCCUG AUGGUAGACG CCGGGGUGTT TTT 33
(2) INFORMATION FOR SEQ ID NO:22:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2'-OMethyl (2'-OMe) modified.
   (ix) FEATURE:
      (D) OTHER INFORMATION: Nucleotides at positions 6-9, 12, 15, 19, 21, 22, and 27 are 2'-amino (2'-NH₂) modified.
   (xi) SEQUENCE DESCRIPTION: SEQ ID NO:22:
      TTTTACCCUG AUGGUAGACG CCGGGGUGTT TTT 33
(2) INFORMATION FOR SEQ ID NO:23:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-5, 10-11, 13-14, 16-18, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:23:
      UUUUACCCUG AUGGUAGACG CCGGGGUG 28
(2) INFORMATION FOR SEQ ID NO:24:
   (i) SEQUENCE CHARACTERIZATION:
      (A) LENGTH: 48
      (B) TYPE: nucleic acid
      (C) STRANDEDNESS: single
      (D) TOPOLOGY: linear
   (ii) MOLECULAR TYPE: RNA
   (xi) SEQUENCE DESCRIPTION: SEQ ID NO:24:
      AGCCATTCAC CATGGCCCCT TCCTACGTAT GTTCTGCGGG TGGCTTAC 48
(2) INFORMATION FOR SEQ ID NO:25:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:25:
      UUUUACCCUG AUGGUAGACG CCGGGGUG 28
(2) INFORMATION FOR SEQ ID NO:26:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 are 2'-OMethyl (2'-OMe) modified..
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:26:
      UUUUACCCUG AUGGUAGACG CCGGGGUG 28
(2) INFORMATION FOR SEQ ID NO:27:
   (i) SEQUENCE CHARACTERIZATION:
      (A) LENGTH: 29
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:27:
      UUUUACCCUG AUGGUAGACG CCGGGGUGT 29
(2) INFORMATION FOR SEQ ID NO:28:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothiate bond.
   (ix) FEATURE:
      (D) OTHER INFORMATION: Nucleotides at positions 5, 8-9, 11, 14, 16, 18, 23, 26, and 28 are 2'-OMethyl (2'OMO) modified.
   (ix) FEATURE:
      (D) OTHER INFORMATION: Nucleotides at positions 6-7, 10, 12, 17, 21-22, 24-25, and 27 are 2'-amino (2'-NH₂) modified.
   (xi) SEQUENCE DESCRIPTION: SEQ ID NO:28:
      TTTTGUCGGU ACGGAGUGGA CCGUCACGTT TTT 33
(2) INFORMATION FOR SEQ ID NO:29:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:29:
      UUULTACCCUG AUGGUAGACG CCGGGGUG 28
(2) INFORMATION FOR SEQ ID NO:30:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
      (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:30:
      UUUUACCCUG AUGGUAGACG CCGGGGUG 28

### SEQUENCE LISTING

(1) GENERAL INFORMATION:
   (i) APPLICANT: LARRY GOLD, PAUL G. SCHMIDT, NEBOJSA JANJIC
   (ii) TITLE OF INVENTION: NUCLEIC ACID LIGAND COMPLEXES
   (iii) NUMBER OF SEQUENCES: 30
   (iv) CORRESPONDENCE ADDRESS:
      (A) ADDRESSEE: Swanson and Bratschun, L.L.C.
      (B) STREET: 8400 East Prentice Avenue, Suite #200
      (C) CITY: Denver
      (D) STATE: Colorado
      (E) COUNTRY: USA
      (F) ZIP: 80111
   (v) COMPUTER READABLE FORM:
      (A) MEDIUM TYPE: Diskette, 3.50 inch, 1.44 Mb storage
      (B) COMPUTER: IBM compatible
      (C) OPERATING SYSTEM: MS-DOS
      (D) SOFTWARE: WordPerfect 8.0
   (vi) CURRENT APPLICATION DATA:
      (A) APPLICATION NUMBER: 08/945,604
      (B) FILING DATE: 28-OCTOBER-1997
      (C) CLASSIFICATION:
   (vii)PRIOR APPLICATION DATA:
      (A) APPLICATION NUMBER: 08/434,465
      (B) FILING DATE: 04-MAY-1995
   (vii)PRIOR APPLICATION DATA:
      (A) APPLICATION NUMBER: 08/464,443
      (B) FILING DATE: 05-JUNE-1995
   (viii) ATTORNEY/AGENT INFORMATION:
      (A) NAME: BARRY J. SWANSON
      (B) REGISTRATION NUMBER: 33,215
      (C) REFERENCE/DOCKET NUMBER: NEX29C/US
   (ix) TELECOMMUNICATION INFORMATION:
      (A) TELEPHONE: (303) 793-3333
      (B) TELEFAX: (303) 793-3433
(2) INFORMATION FOR SEQ ID NO:1:
   (i) SEQUENCE CHARACTERIZATION:
      (A) LENGTH: 23
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:1:
      TTTTTTTTGT CAGGCACCAT CCC 23
(2) INFORMATION FOR SEQ ID NO:2:
   (i) SEQUENCE CHARACTERIZATION:
      (A) LENGTH: 25
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:2:
      CCCCACTGAA GCACCTTTTT TTTTT 25
(2) INFORMATION FOR SEQ ID NO:3:
   (i) SEQUENCE CHARACTERIZATION:
      (A) LENGTH: 36
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:3:
      CAGTCCGTGG TAGGGCAGGT TGGGGTGACT TCGTGG 36
(2) INFORMATION FOR SEQ ID NO:4:
   (i) SEQUENCE CHARACTERIZATION:
      (A) LENGTH: 53
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides 49-53 are bound by a phosphorothioate linkage
   (xi) SEQUENCE DESCRIPTION: SEQ ID NO:4:
      ATCCGCCTGA TTAGCGATAC TCAGAAGGAT AAACTGTCCA GAACTTGGAT 50 TTT 53
(2) INFORMATION FOR SEQ ID NO:5:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide 39 is an inverted-orientation (3'3'linkage) phosphoramidite
   (xi) SEQUENCE DESCRIPTION: SEQ ID NO:5:
      CAGTCCGTGG TAGGGCAGGT TGGGGTGACT TCGTGGAATT 40
(2) INFORMATION FOR SEQ ID NO:6:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides 37 and 38 are bound by a phosphorothioate bond
      (xi) SEQUENCE DESCRIPTION: SEQ ID NO:6:
         CAGTCCGTGG TAGGGCAGGT TGGGGTGACT TCGTGGAA 38
(2) INFORMATION FOR SEQ ID NO:7:
   (i) SEQUENCE CHARACTERIZATION:
      (A) LENGTH: 39
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:7:
      CAGTCCGTGG TAGGGCAGGT TGGGGTGACT TCGTGGAAT 39
(2) INFORMATION FOR SEQ ID NO:8:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 39 is an inverted-orientation (3'3' linkage) phosphoramidite
   (xi) SEQUENCE DESCRIPTION: SEQ ID NO:8:
      CAGTCCGTGG TAGGGCAGGT TGGGGTGACT TCGTGGAATT 40
(2) INFORMATION FOR SEQ ID NO:9:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
   (ix) FEATURE:
      (D) OTHER INFORMATION: Nucleotide number 37 is an inverted-orientation (3'3' linkage) phosphoramidite
   (xi) SEQUENCE DESCRIPTION: SEQ ID NO:9:
      CAGTCCGTGG TAGGGCAGGT TGGGGTGACT TCGTGGTT 38
(2) INFORMATION FOR SEQ ID NO:10:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:10:
      GCGGGGCTAC GTACCGGGGC TTTGTAAAAC CCCGC 35
(2) INFORMATION FOR SEQ ID NO:11:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:11:
      GCGGGGCTAC GTACCGGGGC TTTGTAAAAC CCCGC 35
(2) INFORMATION FOR SEQ ID NO:12:
   (i) SEQUENCE CHARACTERIZATION:
      (A) LENGTH: 40
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 39 is an inverted orientation phosphoramidite

      (xi) SEQUENCE DESCRIPTION: SEQ ID NO:12:
      TCAGTCCGTG GTAGGGCAGG TTGGGGTGAC TTCGTGGATT 40
(2) INFORMATION FOR SEQ ID NO:13:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide 37 is an inverted orientation (3'3' linkage) phosphoramidite
   (xi) SEQUENCE DESCRIPTION: SEQ ID NO: 13:
      CAGTCCGTGG TAGGGCAGGT TGGGGTGACT TCGTGGTT 38
(2) INFORMATION FOR SEQ ID NO:14:
   (i) SEQUENCE CHARACTERIZATION:
      (A) LENGTH: 35
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO: 14:
      GCGGGGCTAC GTACCGGGGC TTTGTAAAAC CCCGC 35
(2) INFORMATION FOR SEQ ID NO:15:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotide number 37 is an inverted orientation (3'3' linkage) phosphoramidite
   (xi) SEQUENCE DESCRIPTION: SEQ ID NO:15:
      TCAGTCCGTG GTAGGGCAGG TTGGGGTGAC TTCGTGTT 38
(2) INFORMATION FOR SEQ ID NO:16:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2-OMethyl (2'-OMe) modified.
   (ix) FEATURE:
      (D) OTHER INFORMATION: Nucleotides at positions 6-9, 12, 15, 19, 21, 22, and 27 are 2'-amino (2'-NH₂) modified.
   (xi) SEQUENCE DESCRIPTION: SEQ ID NO:16:
      TTTTACCCUG AUGGUAGACG CCGGGGUGTT TTT 33
(2) INFORMATION FOR SEQ ID NO:17:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5-28 are 2'-OMethyl (2'-OMe) modified.
   (xi) SEQUENCE DESCRIPTION: SEQ ID NO:17:
      TTTTACCCUG AUGGUAGACG CCGGGGUGTT TTT 33
(2) INFORMATION FOR SEQ ID NO:18:
   (i) SEQUENCE CHARACTERIZATION:
      (A) LENGTH: 38
      (B) TYPE: nucleic acid
      (C) STRANDEDNESS: single
      (D) TOPOLOGY: linear
   (ii) MOLECULAR TYPE: DNA
   (xi) SEQUENCE DESCRIPTION: SEQ ID NO:18:
      CAGTCCGTGG TAGGGCAGGT TGGGGTGACT TCGTGGAA 38
(2) INFORMATION FOR SEQ ID NO:19:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2'-OMethyl(2'-OMe) modified.
   (ix) FEATURE:
      (D) OTHER INFORMATION: Nucleotides at positions 6-9, 12, 15, 19, 21, 22 and 27 are 2'-amino (2'-NH₂) modified.
   (xi) SEQUENCE DESCRIPTION: SEQ ID NO:19:
      TTTTACCCUG AUGGUAGACG CCGGGGUGTT TTT 33
(2) INFORMATION FOR SEQ ID NO:20:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: DNA
   (ix) FEATURE:
      (D) OTHER INFORMATION: All nucleotides are 2'-OMethyl (2'-OMe) modified.
   (xi) SEQUENCE DESCRIPTION: SEQ ID NO:20:
      UUUUACCCUG AUGGUAGACG CCGGGGUG 28
(2) INFORMATION FOR SEQ ID NO: 21:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond
   (ix) FEATURE:
      (D) OTHER INFORMATION: Nucleotides at positions 5, 13, 16, 17, 20, 23-26, and 28 are 2'-OMethyl (2'-OMe) modified
   (ix) FEATURE:
      (D) OTHER INFORMATION: Nucleotides at positions 6-9, 12, 15, 19, 21, 22 and 27 are 2'-amino (2'-NH₂) modified
   (xi) SEQUENCE DESCRIPTION: SEQ ID NO: 21:
      TTTTACCCUG AUGGUAGACG CCGGGGUGTT TTT 33
(2) INFORMATION FOR SEQ ID NO:22:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothioate bond.
   (ix) FEATURE:
      (D) OTHER INFORMATION: Nucleotides at positions 5, 13, 16, 17, 20, 23-26, and 28 are 2'-OMethyl (2'-OMe) modified.
   (ix) FEATURE:
      (D) OTHER INFORMATION: Nucleotides at positions 6-9, 12, 15, 19, 21, 22, and 27 are 2'-amino (2'-NH₂) modified.
   (xi) SEQUENCE DESCRIPTION: SEQ ID NO:22:
      TTTTACCCUG AUGGUAGACG CCGGGGUGTT TTT 33
(2) INFORMATION FOR SEQ ID NO:23:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-5, 10-11, 13-14, 16-18, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:23:
      UUUUACCCUG AUGGUAGACG CCGGGGUG 28
(2) INFORMATION FOR SEQ ID NO:24:
   (i) SEQUENCE CHARACTERIZATION:
      (A) LENGTH: 48
      (B) TYPE: nucleic acid
      (C) STRANDEDNESS: single
      (D) TOPOLOGY: linear
   (ii) MOLECULAR TYPE: RNA
   (xi) SEQUENCE DESCRIPTION: SEQ ID NO:24:
      AGCCATTCAC CATGGCCCCT TCCTACGTAT GTTCTGCGGG TGGCTTAC 48
(2) INFORMATION FOR SEQ ID NO:25:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:25:
      UUUUACCCUG AUGGUAGACG CCGGGGUG 28
(2) INFORMATION FOR SEQ ID NO:26:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:26:
      UUUUACCCUG AUGGUAGACG CCGGGGUG 28
(2) INFORMATION FOR SEQ ID NO:27:
   (i) SEQUENCE CHARACTERIZATION:
      (A) LENGTH: 29
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:27:
      UUUUACCCUG AUGGUAGACG CCGGGGUGT 29
(2) INFORMATION FOR SEQ ID NO:28:
   (i) SEQUENCE CHARACTERIZATION:
      (A) LENGTH: 33
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4 and 29-33 are bound by a phosphorothiate bond.
   (ix) FEATURE:
      (D) OTHER INFORMATION: Nucleotides at positions 5, 8-9, 11, 14, 16, 18, 23, 26, and 28 are 2'-OMethyl (2'OMe) modified.
   (ix) FEATURE:
      (D) OTHER INFORMATION: Nucleotides at positions 6-7, 10, 12, 17, 21-22, 24-25, and 27 are 2'-amino (2'-NH₂) modified.
   (xi) SEQUENCE DESCRIPTION: SEQ ID NO:28:
      TTTTGUCGGU ACGGAGUGGA CCGUCACGTT TTT 33
(2) INFORMATION FOR SEQ ID NO:29:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:29:
      UUUUACCCUG AUGGUAGACG CCGGGGUG 28
(2) INFORMATION FOR SEQ ID NO:30:
   (i) SEQUENCE CHARACTERIZATION:
      (A) LENGTH: 28
      (B) TYPE: nucleic acid
      (C) STRANDEDNESS: single
      (D) TOPOLOGY: linear
   (ii) MOLECULAR TYPE: RNA
   (ix) FEATURE:
      (D) OTHER INFORMATION: Nucleotides at positions 1-4, 13, 16-17, 20, 23-26, and 28 are 2'-OMethyl (2'-OMe) modified.
   (ix) FEATURE:
      (D) OTHER INFORMATION: Nucleotides at positions 6-9, 12, 15, 19, 21-22, and 27 are 2'-Fluoro (2'-F) modified.
   (xi) SEQUENCE DESCRIPTION: SEQ ID NO:30:
      UUUUACCCUG AUGGUAGACG CCGGGGUG 28

## Description

### FIELD OF THE INVENTION

This invention relates to a method for preparing a therapeutic or diagnostic Complex comprised of a Nucleic Acid Ligand and a Non-Immunogenic, High Molecular Weight Compound by identifying a Nucleic Acid Ligand by SELEX methodology and associating the Nucleic Acid Ligand with a Non-Immunogenic, High Molecular Weight Compound. The invention further relates to improving the pharmacokinetic properties of a Nucleic Acid Ligand by associating the Nucleic Acid Ligand to a Non-Immunogenic, High Molecular Weight Compound to form a Complex. The invention also includes complexes comprising one Nucleic Acid Ligand in association with a Non-Immunogenic, High Molecular Weight Compound.

### BACKGROUND OF THE INVENTION

### A. SELEX

The dogma for many years was that nucleic acids had primarily an informational role. Through a method known as Systematic Evolution of Ligands by Exponential enrichment, termed SELEX, it has become clear that nucleic acids have three dimensional structural diversity not unlike proteins. SELEX is a method for the *in vitro* evolution of nucleic acid molecules with highly specific binding to target molecules and is described in United States Patent Application Serial No. 07/536,428, filed June 11, 1990, entitled "Systematic Evolution of Ligands by Exponential Enrichment", now abandoned. United States Patent Application Serial No. 07/714,131, filed June 10, 1991, entitled "Nucleic Acid Ligands", now United States Patent No. 5,475,096, United States Patent Application Serial No. 07/931,473, filed August 17, 1992, entitled "Nucleic Acid Ligands", now United States Patent No. 5,270,163 (see also PCT/US91/04078) . Each of these applications, collectively referred to herein as the SELEX Patent Applications, describes a fundamentally novel method for making a Nucleic Acid Ligand to any desired target molecule. The SELEX process provides a class of products which are referred to as Nucleic Acid Ligands, each ligand having a unique sequence, and which has the property of binding specifically to a desired target compound or molecule. Each SELEX-identified Nucleic Acid Ligand is a specific ligand of a given target compound or molecule. SELEX is based on the unique insight that Nucleic Acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size or composition can serve as targets.

The SELEX method involves selection from a mixture of candidate oligonucleotides and step-wise iterations of binding, partitioning and amplification, using the same general selection scheme, to achieve virtually any desired criterion of binding affinity and selectivity. Starting from a mixture of Nucleic Acids, preferably comprising a segment of randomized sequence, the SELEX method includes steps of contacting the mixture with the target under conditions favorable for binding, partitioning unbound Nucleic Acids from those Nucleic Acids which have bound specifically to target molecules, dissociating the Nucleic Acid-target complexes, amplifying the Nucleic Acids dissociated from the Nucleic Acid-target complexes to yield a ligand-enriched mixture of Nucleic Acids, then reiterating the steps of binding, partitioning, dissociating and amplifying through as many cycles as desired to yield highly specific high affinity Nucleic Acid Ligands to the target molecule.

It has been recognized by the present inventors that the SELEX method demonstrates that Nucleic Acids as chemical compounds can form a wide array of shapes, sizes and configurations, and are capable of a far broader repertoire of binding and other functions than those displayed by Nucleic Acids in biological systems.

The present inventors have recognized that SELEX or SELEX-like processes could be used to identify Nucleic Acids which can facilitate any chosen reaction in a manner similar to that in which Nucleic Acid Ligands can be identified for any given target. In theory, within a Candidate Mixture of approximately 10¹³ to 10¹⁸ Nucleic Acids, the present inventors postulate that at least one Nucleic Acid exists with the appropriate shape to facilitate each of a broad variety of physical and chemical interactions.

The basic SELEX method has been modified to achieve a number of specific objectives. For example, United States Patent Application Serial No. 07/960,093, filed October 14, 1992, entitled "Method for Selecting Nucleic Acids on the Basis of Structure." describes the use of SELEX in conjunction with gel electrophoresis to select Nucleic Acid molecules with specific structural characteristics, such as bent DNA. United States Patent Application Serial No. 08/123,935, filed September 17, 1993, entitled "Photoselection of Nucleic Acid Ligands," describes a SELEX based method for selecting Nucleic Acid Ligands containing photoreactive groups capable of binding and/or photocrosslinking to and/or photoinactivating a target molecule. United States Patent Application Serial No. 08/134,028, filed October 7, 1993, entitled "High-Affinity Nucleic Acid Ligands That Discriminate Between Theophylline and Caffeine," describes a method for identifying highly specific Nucleic Acid Ligands able to discriminate between closely related molecules, which can be non-peptidic, termed Counter-SELEX. United States Patent Application Serial No. 08/143,564, filed October 25, 1993, entitled "Systematic Evolution of Ligands by Exponential Enrichment: Solution SELEX," describes a SELEX-based method which achieves highly efficient partitioning between oligonucleotides having high and low affinity for a target molecule.

The SELEX method encompasses the identification of high-affinity Nucleic Acid Ligands containing modified nucleotides conferring improved characteristics on the ligand, such as improved *in vivo* stability or improved delivery characteristics. Examples of such modifications include chemical substitutions at the ribose and/or phosphate and/or base positions. SELEX-identified Nucleic Acid Ligands containing modified nucleotides are described in United States Patent Application Serial No. 08/117,991, filed September 8, 1993, entitled "High Affinity Nucleic Acid Ligands Containing Modified Nucleotides," that describes oligonucleotides containing nucleotide derivatives chemically modified at the 5- and 2'-positions of pyrimidines. United States Patent Application Serial No. 08/134,028, *supra,* describes highly specific Nucleic Acid Ligands containing one or more nucleotides modified with 2'-amino (2'-NH₂), 2'-fluoro (2'-F), and/or 2'-O-methyl (2'-OMe). United States Patent Application Serial No. 08/264,029, filed June 22, 1994, entitled "Novel Method of Preparation of 2' Modified Pyrimidine Intramolecular Nucleophilic Displacement", describes oligonucleotides containing various 2'-modified pyrimidines.

The SELEX method encompasses combining selected oligonucleotides with other selected oligonucleotides and non-oligonucleotide functional units as described in United States Patent Application Serial No. 08/284,063, filed August 2, 1994, entitled "Systematic Evolution of Ligands by Exponential Enrichment: Chimeric SELEX" and United States Patent Application Serial No. 08/234,997, filed April 28, 1994, entitled "Systematic Evolution of Ligands by Exponential Enrichment: Blended SELEX," respectively. These applications allow the combination of the broad array of shapes and other properties, and the efficient amplification and replication properties, of oligonucleotides with the desirable properties of other molecules.

A few instances have been reported where researchers have attached antisense oligonucleotides to Non-Immunogenic, High Molecular Weight Compounds. Antisense oligonucleotides, however, are only effective as intracellular agents. Antisense oligodeoxyribonucleotides targeted to the epidermal growth factor (EGF) receptor have been encapsulated into Liposomes linked to folate via a polyethylene glycol spacer (folate-PEG-Liposomes) and delivered into cultured KB cells via folate receptor-mediated endocytosis (Wang *et al.* (1995) 92:3318-3322).

### SUMMARY OF THE INVENTION

The present invention provides a method for preparing a therapeutic or diagnostic Complex comprised of a Nucleic Acid Ligand and a Non-Immunogenic, High Molecular Weight Compound by the method comprising identifying a Nucleic Acid Ligand from a Candidate Mixture of Nucleic Acids where the Nucleic Acid is a ligand of a given target by the method of (a) contacting the Candidate Mixture of Nucleic Acids with the target, (b) partitioning between members of said Candidate Mixture on the basis of affinity to the target, and c) amplifying the selected molecules to yield a mixture of Nucleic Acids enriched for Nucleic Acid sequences with a relatively higher affinity for binding to the target, and associating said identified Nucleic Acid Ligand with a Non-Imnunogenic, High Molecular Weight Compound.

This disclosure provides a method for improving the cellular uptake of a Nucleic Acid Ligand having an intracellular SELEX Target by associating the Nucleic Acid Ligand with a Non-Immunogenic, High Molecular Weight Compound to form a Complex comprised of a Nucleic Acid Ligand and a Non-Immunogenic, High Molecular Weight Compound and administering the Complex to a patient.

This disclosure provides a method for improving the pharmacokinetic properties of a Nucleic Acid Ligand by associating the Nucleic Acid Ligand to a Non-Immunogenic, High Molecular Weight Compound to form a Complex comprised of a Nucleic Acid Ligand and a Non-Immunogenic, High Molecular Weight Compound and administering the Complex to a patient.

It is an object of the present invention to provide Complexes comprising one Nucleic Acid Ligand in association with a Non-Immunogenic, High Molecular Weight Compound and methods for producing the same. It is a further object of the invention to provide one Nucleic Acid Ligand in association with a Non-Immunogenic, High Molecular Weight Compound with Improved Pharmacokinetic Properties. The Non-Immunogenic, High Molecular Weight Compound is PEG. The Nucleic Acid Ligand is identified according to the SELEX method.

In embodiments of the invention directed to Complexes comprising PEG in association with a Nucleic Acid Ligand, the Nucleic Acid Ligand can serve in a targeting capacity.

In embodiments where the Nucleic Acid Ligand of the Complex serves in a targeting capacity, the Complex can incorporate or have associated with it therapeutic or diagnostic agents. In one embodiment, the therapeutic agent is a drug.

These and other objects, as well as the nature, scope and utilization of this invention, will become readily apparent to those skilled in the art from the following description and the appended claims.

### BRIEF DESCRIPTION OF THE FIGURES

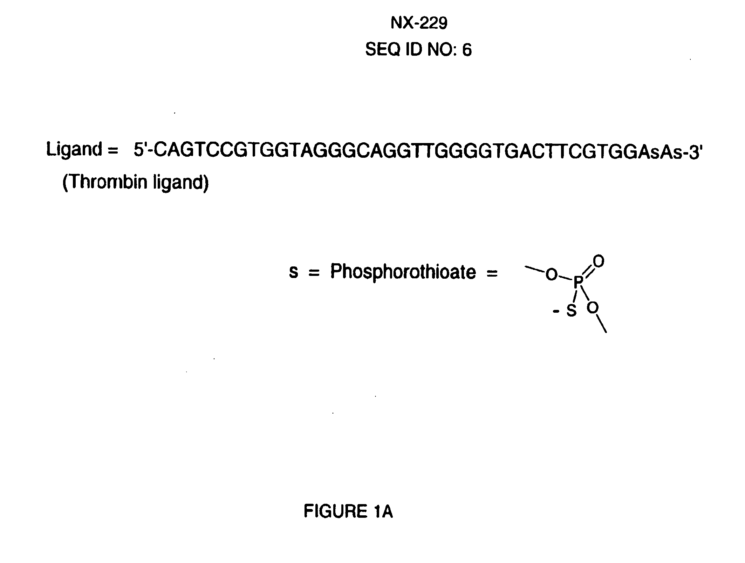

Figures 1A-1Y show the molecular descriptions of NX229, NX232, NX253, NX256, 225T3, 225T3N, T-P4, NX-256-PEG-20,000, 225T3N-PEG-3400, T-P4-PEG-(20,000 or 10,000), NX268, NX191, JW966, NX278, JW986, NX213, NX244, JW1130, NX287, JW1336-20K PEG, JW1379, JW1380, scNX278, JW986-PEG-(10,000, 20,000, or 40,000), and JW1336 (SEQ ID NOS:6-30). A lower case letter preceding a nucleotide indicates the following: m=2'-O-Methyl, a=2'-amino, r=ribo, and f=2'-fluoro. No letter preceding a nucleotide indicates a deoxyribonucleotide (2'H). An S following a nucleotide denotes a backbone modification consisting of a phosphorothioate internucleoside linkage.
Figure 2 summarizes the data for the plasma concentration of NX229, NX232, NX253, NX253 + Liposome, and NX256-PEG20K as a function of time following the bolus injection.
Figure 3 summarizes the data for the plasma concentration of NX213 (SEQ ID NO:21), NX268 (SEQ ID NO:16), NX278 (SEQ ID NO:19), NX278 + liposome, JW986 (SEQ ID NO:20), NX213 liposome encapsulated, and NX244 (SEQ ID NO:22) as a function of time following the bolus injection.
Figure 4 summarizes the data for the plasma concentration of JW966 (SEQ ID NO:18) and JW966 + liposome as a function of time following the bolus injection.
Figure 5 summarizes the data for the plasma concentration of NX268 (SEQ ID NO:16) and NX268 + liposome as a function of time following the bolus injection.
Figure 6 summarizes the data for the plasma concentration of NX191 (SEQ ID NO:17), JW986 + PEG20K, PEG40K, and PEG10K (SEQ ID NO:29) as a function of time following the bolus injection.
Figure 7 summarizes the data for the plasma concentration of JW986 + PEG20K (SEQ ID NO:29) and JW1130 (SEQ ID NO:23) as a function of time following the bolus injection.
Figure 8 summarizes the data for the plasma concentration of NX287 + PEG40K (SEQ ID NO:24) and NX256 (SEQ ID NO:9) as a function of time following the bolus injection.
Figure 9 summarizes the data for the plasma concentration of JW1130 (SEQ ID NO:23), 1136-PEG20K (SEQ ID NO:25), 1336 (SEQ ID NO:30), and 1379/80 (SEQ ID NOS:26-27) as a function of time following the bolus injection.

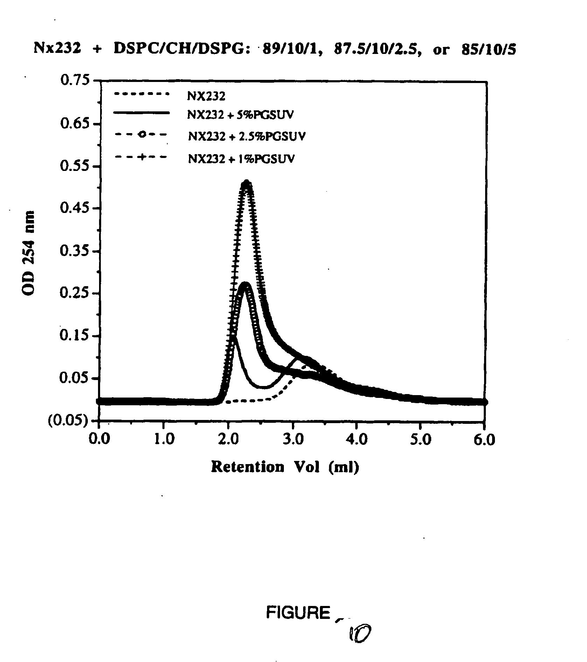

Figure 10 shows the chromatograms for NX232 (SEQ ID NO:7), NX232 + 1% PGSUV, NX232 + 2.5% PGSUV, and NX232 + PGSUV.

### DETAILED DESCRIPTION OF THE INVENTION

### DEFINITIONS:

"**Covalent Bond**" is the chemical bond formed by the sharing of electrons.

"**Non-Covalent Interactions**" are means of holding together molecular entities by interactions other than Covalent Bonds including ionic interactions and hydrogen bonds.

"**Lipid Constructs**," for purposes of this disclosure, are structures containing lipids, phospholipids, or derivatives thereof comprising a variety of different structural arrangements which lipids are known to adopt in aqueous suspension. These structures include, but are not limited to, Lipid Bilayer Vesicles, micelles, Liposomes, emulsions, lipid ribbons or sheets, and may be complexed with a variety of drugs and adjuvants which are known to be pharmaceutically acceptable. Common adjuvants include cholesterol and alpha-tocopherol, among others. The Lipid Constructs may be used alone or in any combination which one skilled in the art would appreciate to provide the characteristics desired for a particular application. In addition, the technical aspects of Lipid Constructs and Liposome formation are well known in the art and any of the methods commonly practiced in the field may be used.

"**Lipophilic Compounds**" are compounds which have the propensity to associate with or partition into lipid and/or other materials or phases with low dielectric constants, including structures that are comprised substantially of lipophilic components. Lipophilic Compounds include Lipid Constructs as well as non-lipid containing compounds that have the propensity to associate with lipid (and/or other materials or phases with low dielectric constants). Cholesterol, phospholipid, and dialkyl glycerol are further examples of Lipophilic Compounds.

"**Complex**" as used herein describes the molecular entity formed by the association of a Nucleic Acid Ligand with a Non-immunogenic, High Molecular Weight Compound. The association can be through either Covalent Bonds or Non-Covalent Interactions.

"**Nucleic Acid Ligand**" as used herein is a non-naturally occurring Nucleic Acid having a desirable action on a SELEX Target. A desirable action includes, but is not limited to, binding of the SELEX Target, catalytically changing the SELEX Target, reacting with the SELEX Target in a way which modifies/alters the SELEX Target or the functional activity of the SELEX Target, covalently attaching to the SELEX Target as in a suicide inhibitor, facilitating the reaction between the Target and another molecule. In the preferred embodiment, the action is specific binding affinity for a Target molecule, such Target molecule being a three dimensional chemical structure other than a polynucleotide that binds to the Nucleic Acid Ligand through a mechanism which depends on Watson/Crick base pairing or triple helix binding, wherein the Nucleic Acid Ligand is not a Nucleic Acid having the known physiological function of being bound by the Target molecule. In preferred embodiments of the invention, the Nucleic Acid Ligand of the Complexes of the invention are identified by the SELEX methodology. Nucleic Acid Ligands include Nucleic Acids that are identified from a Candidate Mixture of Nucleic Acids, said Nucleic Acid being a ligand of a given Target, by the method comprising a) contacting the Candidate Mixture with the Target, wherein Nucleic Acids having an increased affinity to the Target relative to the Candidate Mixture may be partitioned from the remainder of the Candidate Mixture; b) partitioning the increased affinity Nucleic Acids from the remainder of the Candidate Mixture; and c) amplifying the increased affinity Nucleic Acids to yield a ligand-enriched mixture of Nucleic Acids.

"**Candidate Mixture**" is a mixture of Nucleic Acids of differing sequence from which to select a desired ligand. The source of a Candidate Mixture can be from naturally-occurring Nucleic Acids or fragments thereof, chemically synthesized Nucleic Acids, enzymatically synthesized Nucleic Acids or Nucleic Acids made by a combination of the foregoing techniques. In a preferred embodiment, each Nucleic Acid has fixed sequences surrounding a randomized region to facilitate the amplification process.

"**Nucleic Acid**" means either DNA, RNA, single-stranded or double-stranded and any chemical modifications thereof. Modifications include, but are not limited to, those which provide other chemical groups that incorporate additional charge, polarizability, hydrogen bonding, electrostatic interaction, and fluxionality to the Nucleic Acid Ligand bases or to the Nucleic Acid Ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil, backbone modifications such as internucleoside phosphorothioate linkages, methylations, unusual base-pairing combinations such as the isobases isocytidine and isoguanidine and the like. Modifications can also include 3' and 5' modifications such as capping.

"**Non-Immunogenic**, **High Molecular Weight Compound**" is a polyethylene glycol compound of approximately 1000 Da or more.

"**Lipid Bilayer Vesicles**" are closed, fluid-filled microscopic spheres which are formed principally from individual molecules having polar (hydrophilic) and non-polar (lipophilic) portions. The hydrophilic portions may comprise phosphato, glycerylphosphato, carboxy, sulfato, amino, hydroxy, choline and other polar groups. Examples of non-polar groups are saturated or unsaturated hydrocarbons such as alkyl, alkenyl or other lipid groups. Sterols (e.g., cholesterol) and other pharmaceutically acceptable adjuvants (including anti-oxidants like alpha-tocopherol) may also be included to improve vesicle stability or confer other desirable characteristics.

"**Liposomes**" are a subset of bilayer vesicles and are comprised principally of phospholipid molecules which contain two hydrophobic tails consisting of long fatty acid chains. Upon exposure to water, these molecules spontaneously align to form a bilayer membrane with the lipophilic ends of the molecules in each layer associated in the center of the membrane and the opposing polar ends forming the respective inner and outer surface of the bilayer membrane. Thus, each side of the membrane presents a hydrophilic surface while the interior of the membrane comprises a lipophilic medium. These membranes when formed are generally arranged in a system of concentric closed membranes separated by interlamellar aqueous phases, in a manner not dissimilar to the layers of an onion, around an internal aqueous space. These multilamellar vesicles (MLV) can be converted into small or unilamellar vesicles (UV), with the application of a shearing force.

"**Cationic Liposome**" is a Liposome that contains lipid components that have an overall positive charge at physiological pH.

"**SELEX"** methodology involves the combination of selection of Nucleic Acid Ligands which interact with a Target in a desirable manner, for example binding to a protein, with amplification of those selected Nucleic Acids. Iterative cycling of the selection/amplification steps allows selection of one or a small number of Nucleic Acids which interact most strongly with the Target from a pool which contains a very large number of Nucleic Acids. Cycling of the selection/amplifcation procedure is continued until a selected goal is achieved. In the present invention, the SELEX methodology can be employed to obtain a Nucleic Acid Ligand to a desirable Target.

The SELEX methodology is described in the SELEX Patent Applications.

"**SELEX Target**" means any compound or molecule of interest for which a ligand is desired. A Target can be a protein (such as VEGF, thrombin, and selectin), peptide, carbohydrate, polysaccharide, glycoprotein, hormone, receptor, antigen, antibody, virus, substrate, metabolite, transition state analog, cofactor, inhibitor, drug, dye, nutrient, growth factor, etc. without limitation. The terms "SELEX Target" and "Target" can be used interchangeably herein. It will be clear from the sentence context whether or not "Target" means "SELEX Target."

"**Target**" means a preselected location in a biological system including tissues, organs, cells, intracellular compartments, extracellular components. The latter include hormones (endocrine paracrine, autocrine), enzymes, neurotransmitters and constituents of physiological cascade phenomena (e.g., blood coagulation, complement, etc.).

"**Improved Pharmacokinetic Properties**" means that the Nucleic Acid Ligand in association with the Non-Immunogenic, High Molecular Weight Compound shows a longer circulation half-life *in vivo* relative to the same Nucleic Acid Ligand not in association with a Non-Immunogenic, High Molecular Weight Compound or other pharmacokinetic benefits such as improved Target to non-Target concentration ratio.

"**Linker**" is a molecular entity that connects two or more molecular entities through covalent or Non-Covalent Interactions.

"Spacer" is a Linker of the size that allows spatial separation of two or more molecular entities in a manner that preserves the functional properties of one or more of the molecular entities.

It is an object of the present invention to provide Complexes comprising one or more Nucleic Acid Ligands in association with a Non-Immunogenic, High Molecular Weight Compound. Such Complexes have one or more of the following advantages over a Nucleic Acid Ligand not in association with a Non-Immunogenic, High Molecular Weight Compound: 1) Improved Pharmacokinetic Properties, 2) improved capacity for intracellular delivery, or 3) improved capacity for targeting.

The Complexes of the present invention may benefit from one, two, or all three of these advantages. The Complexes of the present invention may contain different Nucleic Acid Ligands serving totally different purposes in the Complex.

In an embodiment, the Complex of the present invention is comprised of a Nucleic Acid Ligand covalently attached to polyethylene glycol (PEG). The pharmacokinetic properties of the Complex will be enhanced relative to the Nucleic Acid Ligand alone.

The Non-Immunogenic, High Molecular Weight Compound is covalently bonded with the Nucleic Acid Ligand(s). The polyethylene glycol may be covalently bound to a hydroxyl group or other group at the 5' or 3' terminus of the Nucleic Acid Ligand. Preferably, however, it is bonded to the 5' or 3' hydroxyl group thereof. Attachment of the Nucleic Acid Ligand to other components of the Complex can be done directly or with the utilization of Linkers or Spacers.

One problem encountered in the therapeutic and *in vivo* diagnostic use of Nucleic Acids is that oligonucleotides in their phosphodiester form may be quickly degraded in body fluids by intracellular and extracellular enzymes such as endonucleases and exonucleases before the desired effect is manifest. Certain chemical modifications of the Nucleic Acid Ligand can be made to increase the *in vivo* stability of the Nucleic Acid Ligand or to enhance or to mediate the delivery of the Nucleic Acid Ligand. Modifications of the Nucleic Acid Ligands contemplated in this invention include, but are not limited to, those which provide other chemical groups that incorporate additional charge, polarizability, hydrophobicity, hydrogen bonding, electrostatic interaction, and fluxionality to the Nucleic Acid Ligand bases or to the Nucleic Acid Ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, unusual base-pairing combinations such as the isobases isocytidine and isoguanidine and the like. Modifications can also include 3' and 5' modifications such as capping.

Where the Nucleic Acid Ligands are derived by the SELEX method, the modifications can be pre- or post- SELEX modifications. Pre-SELEX modifications yield Nucleic Acid Ligands with both specificity for their SELEX Target and improved *in vivo* stability. Post-SELEX modifications made to 2'-OH Nucleic Acid Ligands can result in improved *in vivo* stability without adversely affecting the binding capacity of the Nucleic Acid Ligands. The preferred modifications of the Nucleic Acid Ligands of the subject invention are 5' and 3' phosphorothioate capping or 3'3' inverted phosphodiester linkage at the 3' end. For RNA ligands, additional 2' amino (2'-NH₂) modification of some or all of the nucleotides is preferred.

In another aspect of the present invention, the association of the Nucleic Acid Ligand with a Non-Immunogenic, High Molecular Weight Compound, results in Improved Pharmacokinetic Properties (i.e., slower clearance rate) relative to the Nucleic Acid Ligand not in association with a Non-Immunogenic, High Molecular Weight Compound.

A Nucleic Acid Ligand or ligands in association with a or Non-Immunogenic, High Molecular Weight Compound may enhance the intracellular delivery of the Nucleic Acid Ligand(s) over non-associated Nucleic Acid Ligand(s).

In the preferred embodiment, the Nucleic Acid Ligands of the present invention are derived from the SELEX methodology. SELEX is described in U.S. Patent Application Serial No. 07/536,428, entitled Systematic Evolution of Ligands by Exponential Enrichment, now abandoned, U.S. Patent Application Serial No. 07/714,131, filed June 10, 1991, entitled Nucleic Acid Ligands, now United States Patent No. 5,475,096, United States Patent Application Serial No. 07/931,473, filed August 17, 1992, entitled Nucleic Acid Ligands, now United States Patent No. 5,270,163 (see also PCT/US91/04078). These applications are collectively called the SELEX Patent Applications.

The SELEX process provides a class of products which are Nucleic Acid molecules, each having a unique sequence, and each of which has the property of binding specifically to a desired Target compound or molecule. Target molecules are preferably proteins, but can also include among others carbohydrates, peptidoglycans and a variety of small molecules. SELEX methodology can also be used to Target biological structures, such as cell surfaces or viruses, through specific interaction with a molecule that is an integral part of that biological structure.

In its most basic form, the SELEX process may be defined by the following series of steps:
1) A Candidate Mixture of Nucleic Acids of differing sequence is prepared. The Candidate Mixture generally includes regions of fixed sequences (i.e., each of the members of the Candidate Mixture contains the same sequences in the same location) and regions of randomized sequences. The fixed sequence regions are selected either: (a) to assist in the amplification steps described below, (b) to mimic a sequence known to bind to the Target, or (c) to enhance the concentration of a given structural arrangement of the Nucleic Acids in the Candidate Mixture. The randomized sequences can be totally randomized (i.e., the probability of finding a base at any position being one in four) or only partially randomized (e.g., the probability of finding a base at any location can be selected at any level between 0 and 100 percent).
2) The Candidate Mixture is contacted with the selected Target under conditions favorable for binding Between the Target and members of the Candidate Mixture. Under these circumstances, the interaction between the Target and the Nucleic Acids of the Candidate Mixture can be considered as forming Nucleic Acid-target pairs between the Target and those Nucleic Acids having the strongest affinity for the Target.
3) The Nucleic Acids with the highest affinity for the target are partitioned from those Nucleic Acids with lesser affinity to the target. Because only an extremely small number of sequences (and possibly only one molecule of Nucleic Acid) corresponding to the highest affinity Nucleic Acids exist in the Candidate Mixture, it is generally desirable to set the partitioning criteria so that a significant amount of the Nucleic Acids in the Candidate Mixture (approximately 5-50%) are retained during partitioning.
4) Those Nucleic Acids selected during partitioning as having the relatively higher affinity for the target are then amplified to create a new Candidate Mixture that is enriched in Nucleic Acids having a relatively higher affinity for the target.
5) By repeating the partitioning and amplifying steps above, the newly formed Candidate Mixture contains fewer and fewer unique sequences, and the average degree of affinity of the Nucleic Acids to the target will generally increase. Taken to its extreme, the SELEX process will yield a Candidate Mixture containing one or a small number of unique Nucleic Acids representing those Nucleic Acids from the original Candidate Mixture having the highest affinity to the target molecule.

The basic SELEX method has been modified to achieve a number of specific objectives. For example, United States Patent Application Serial No. 07/960,093, filed October 14, 1992, entitled "Method for Selecting Nucleic Acids on the Basis of Structure", describes the use of SELEX in conjunction with gel electrophoresis to select Nucleic Acid molecules with specific structural characteristics, such as bent DNA. United States Patent Application Serial No. 08/123,935, filed September 17, 1993, entitled "Photoselection of Nucleic Acid Ligands" describes a SELEX based method for selecting Nucleic Acid Ligands containing photoreactive groups capable of binding and/or photocrosslinking to and/or photoinactivating a target molecule. United States Patent Application Serial No. 08/134,028, filed October 7, 1993, entitled "High-Affinity Nucleic Acid Ligands That Discriminate Between Theophylline and Caffeine", describes a method for identifying highly specific Nucleic Acid Ligands able to discriminate between closely related molecules, termed Counter-SELEX. United States Patent Application Serial No. 08/143,564, filed October 25, 1993, entitled "Systematic Evolution of Ligands by Exponential Enrichment: Solution SELEX", describes a SELEX-based method which achieves highly efficient partitioning between oligonucleotides having high and low affinity for a target molecule. United States Patent Application Serial No. 07/964,624, filed October 21, 1992, entitled "Methods of Producing Nucleic Acid Ligands" describes methods for obtaining improved Nucleic Acid Ligands after SELEX has been performed. United States Patent Application Serial No. 08/400,440, filed March 8, 1995, entitled "Systematic Evolution of Ligands by EXponential Enrichment: Chemi-SELEX", describes methods for covalently linking a ligand to its target.

The SELEX method encompasses the identification of high-affinity Nucleic Acid Ligands containing modified nucleotides conferring improved characteristics on the ligand, such as improved *in vivo* stability or improved delivery characteristics. Examples of such modifications include chemical substitutions at the ribose and/or phosphate and/or base positions. SELEX-identified Nucleic Acid Ligands containing modified nucleotides are described in United States Patent Application Serial No. 08/117,991, filed September 8, 1993, entitled "High Affinity Nucleic Acid Ligands Containing Modified Nucleotides", that describes oligonucleotides containing nucleotide derivatives chemically modified at the 5- and 2'-positions of pyrimidines. United States Patent Application Serial No. 08/134,028, *supra,* describes highly specific Nucleic Acid Ligands containing one or more nucleotides modified with 2'-amino (2'-NH₂), 2'-fluoro (2'-F), and/or 2'-O-methyl (2'-OMe). United States Patent Application Serial No. 08/264,029, filed June 22, 1994, entitled "Novel Method of Preparation of 2' Modified Pyrimidine Intramolecular Nucleophilic Displacement", describes oligonucleotides containing various 2'-modified pyrimidines.

The SELEX method encompasses combining selected oligonucleotides with other selected oligonucleotides and non-oligonucleotide functional units as described in United States Patent Application Serial No. 08/284,063, filed August 2, 1994, entitled "Systematic Evolution of Ligands by Exponential Enrichment: Chimeric SELEX" and United States Patent Application Serial No. 08/234,997, filed April 28, 1994, entitled "Systematic Evolution of Ligands by Exponential Enrichment: Blended SELEX", respectively. These applications allow the combination of the broad array of shapes and other properties, and the efficient amplification and replication properties, of oligonucleotides with the desirable properties of other molecules. Each of the above described patent applications which describe modifications of the basic SELEX procedure are specifically incorporated by reference herein in their entirety.

SELEX identifies Nucleic Acid Ligands that are able to bind targets with high affinity and with outstanding specificity, which represents a singular achievement that is unprecedented in the field of Nucleic Acids research. These characteristics are, of course, the desired properties one skilled in the art would seek in a therapeutic or diagnostic ligand.

In order to produce Nucleic Acid Ligands desirable for use as a pharmaceutical, it is preferred that the Nucleic Acid Ligand (1) binds to the target in a manner capable of achieving the desired effect on the target; (2) be as small as possible to obtain the desired effect; (3) be as stable as possible; and (4) be a specific ligand to the chosen target. In most situations, it is preferred that the Nucleic Acid Ligand has the highest possible affinity to the target. Additionally, Nucleic Acid Ligands can have facilitating properties.

In co-pending and commonly assigned U.S. Patent Application Serial No. 07/964,624, filed October 21, 1992 ('624), methods are described for obtaining improved Nucleic Acid Ligands after SELEX has been performed.

In embodiments where the Nucleic Acid Ligand(s) can serve in a targeting capacity, the Nucleic Acid Ligands adopt a three dimensional structure that must be retained in order for the Nucleic Acid Ligand to be able to bind its target In addition, the Nucleic Acid Ligand must be properly oriented with respect to the surface of the Complex so that its Target binding capacity is not compromised. This can be accomplished by attaching the Nucleic Acid Ligand at a position that is distant from the binding portion of the Nucleic Acid Ligand. The three dimensional structure and proper orientation can also be preserved by use of a Linker or Spacer as described *supra.*

Any variety of therapeutic or diagnostic agents can be attached or incorporated into the Complex as discussed *supra* for targeted delivery by the Complex.

In other embodiments, the Complex of the present invention is comprised of a Nucleic Acid Ligand attached to a PEG. In this embodiment, the pharmacokinetic properties of the Complex are improved relative to the Nucleic Acid Ligand alone. As discussed *supra*, the association is through Covalent Bonds. Also, as discussed *supra,* PEG may be covalently bound to a variety of positions on the Nucleic Acid Ligand. Where PEG is used, it is preferred that the Nucleic Acid Ligand is bonded to the 5' thiol through a maleimide or vinyl sulfone functionality or via a phosphodiester linkage. In yet further embodiments, a plurality of PEG molecules can be attached to each other.

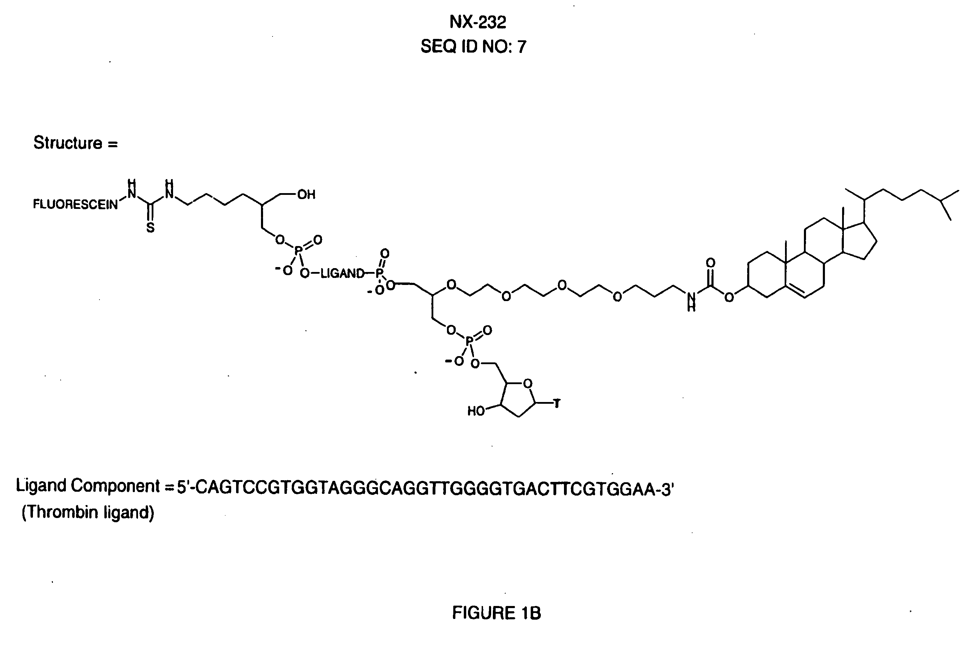

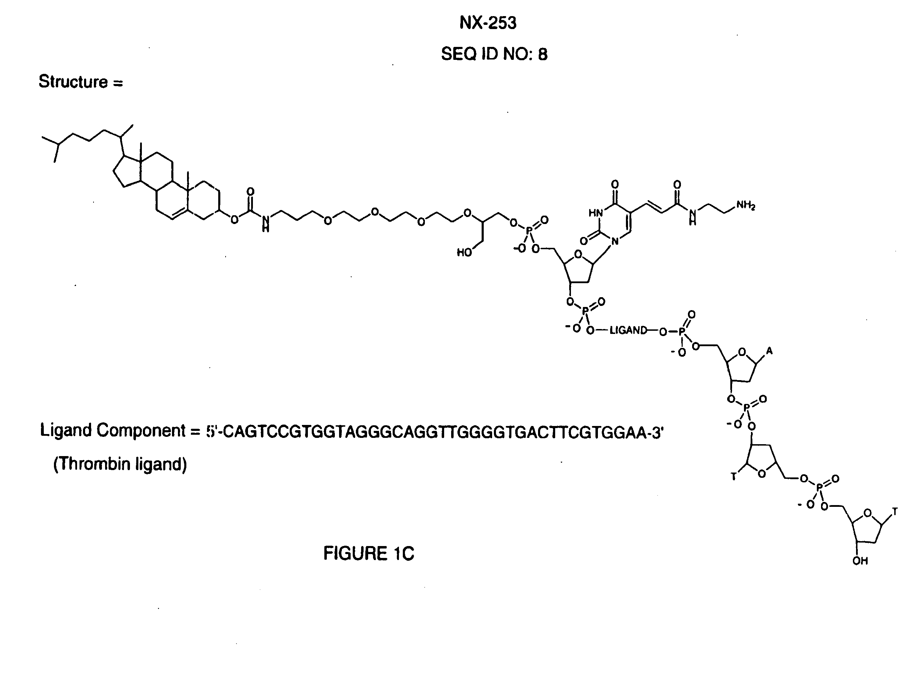

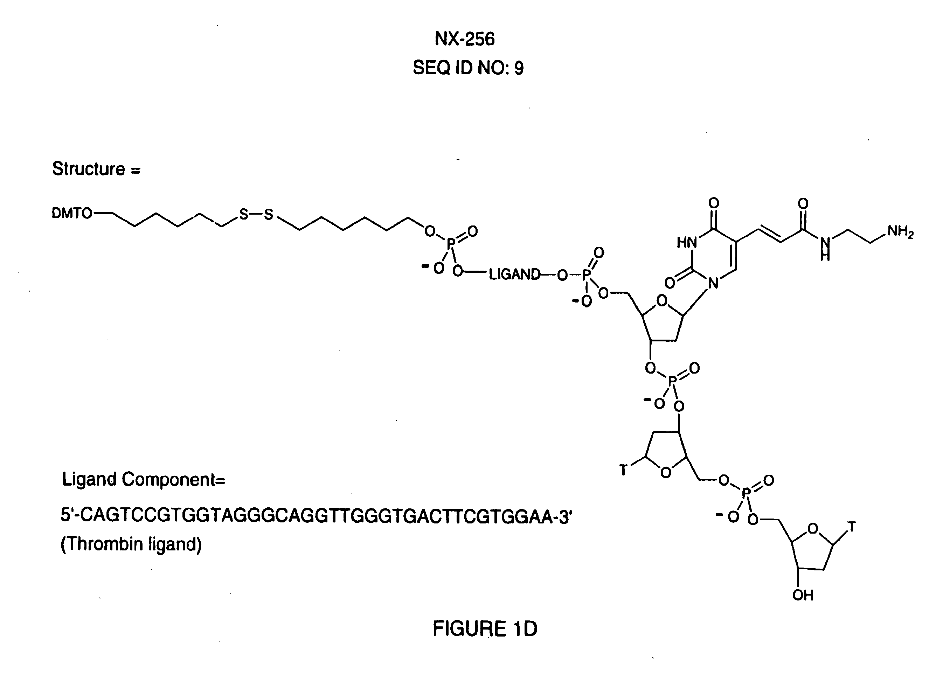

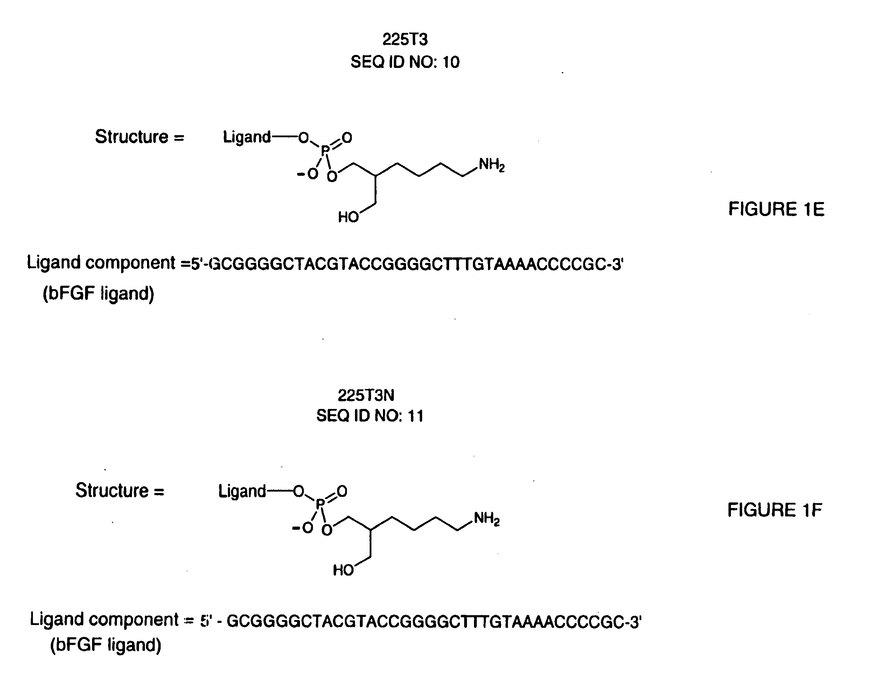

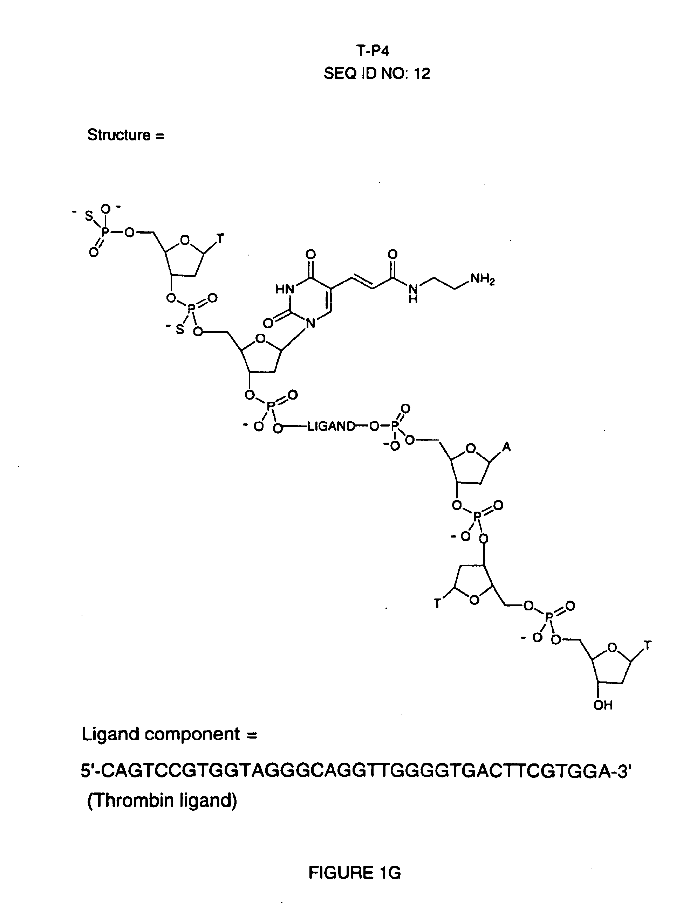

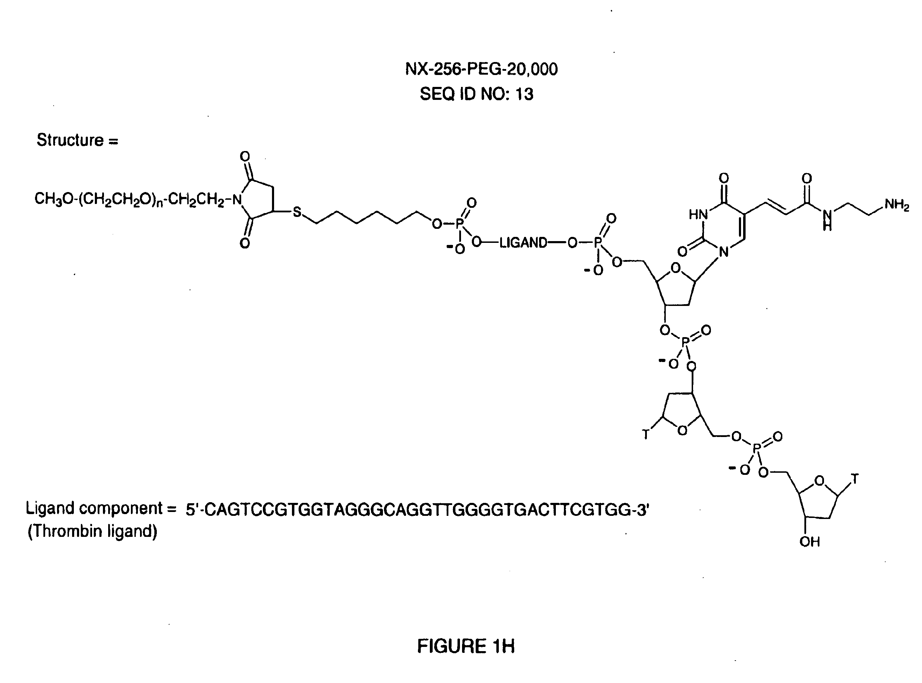

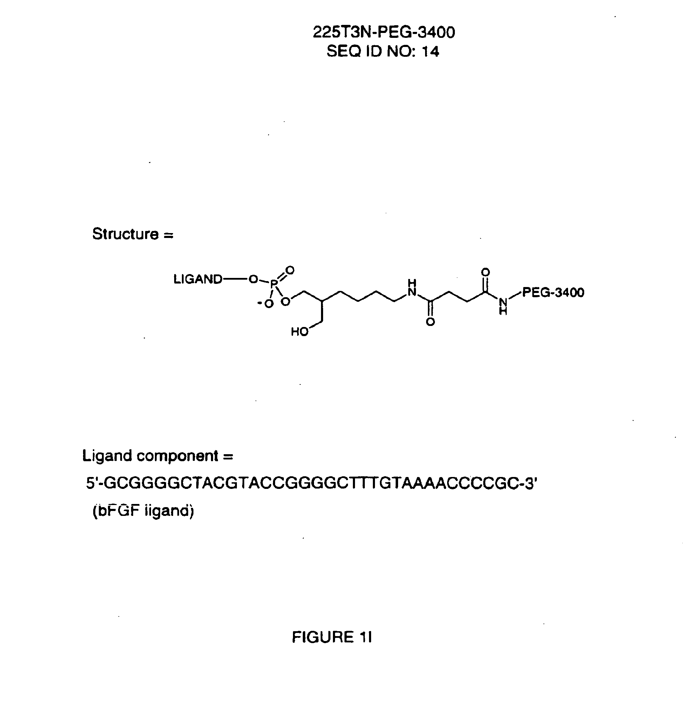

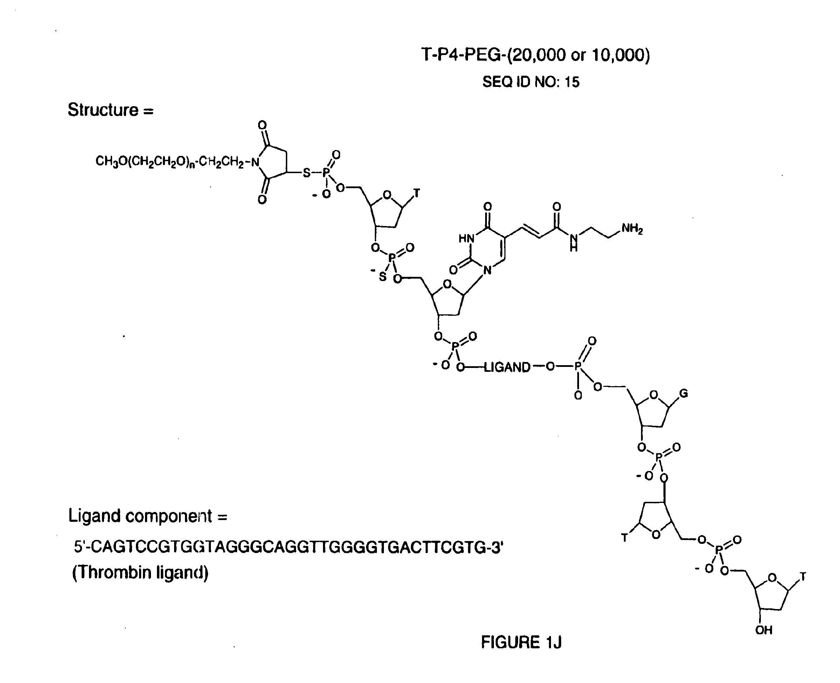

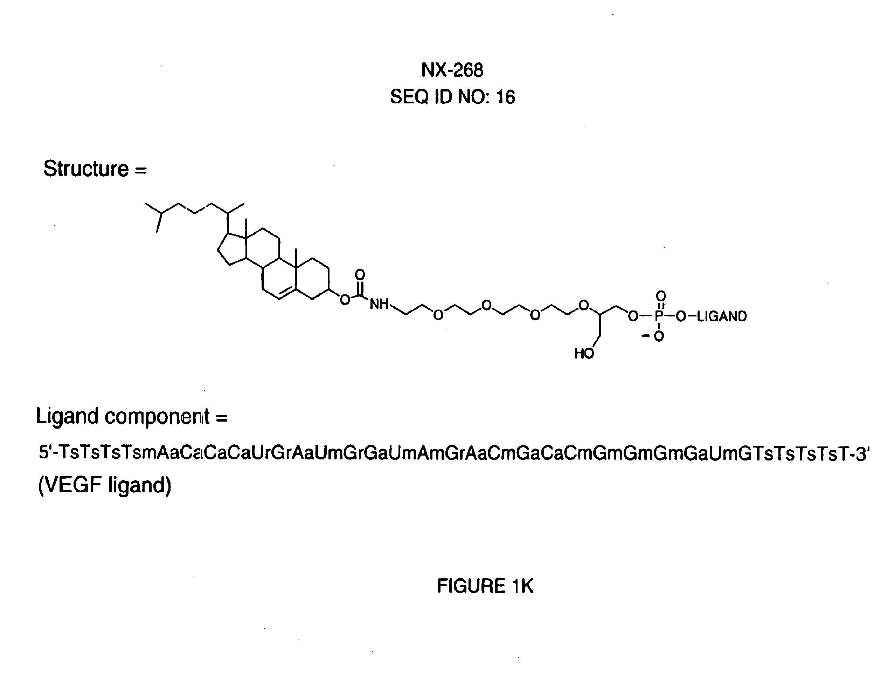

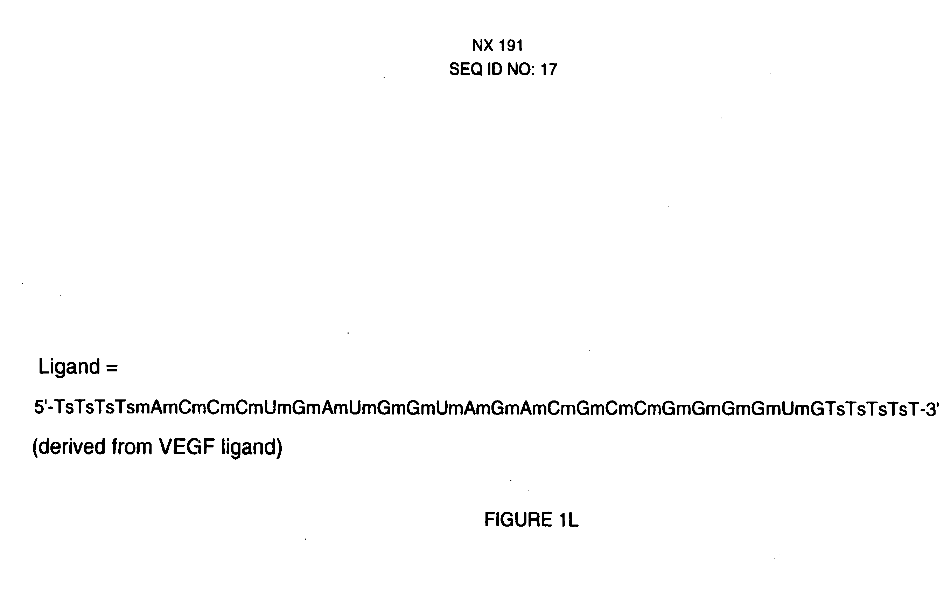

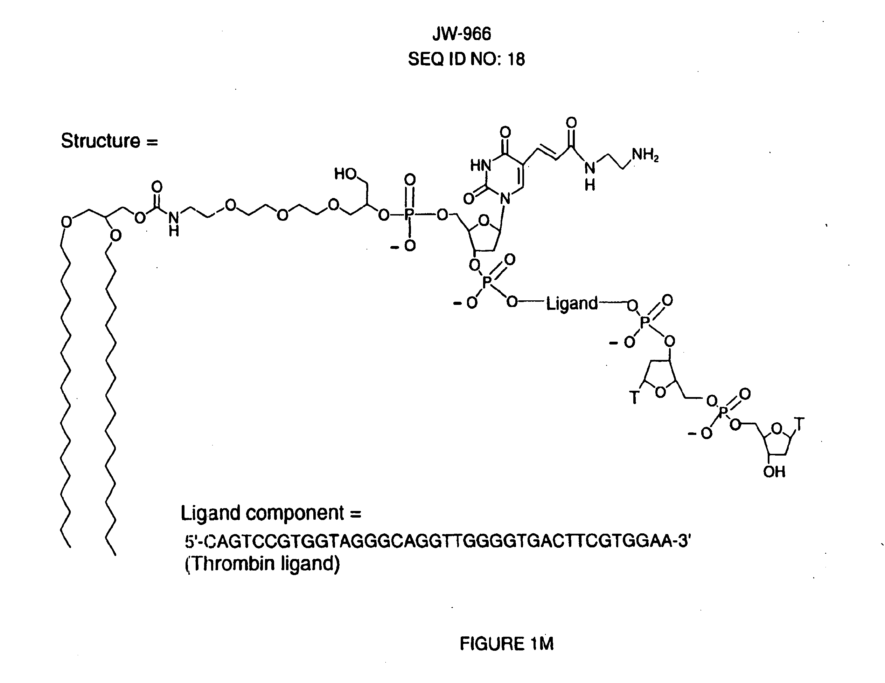

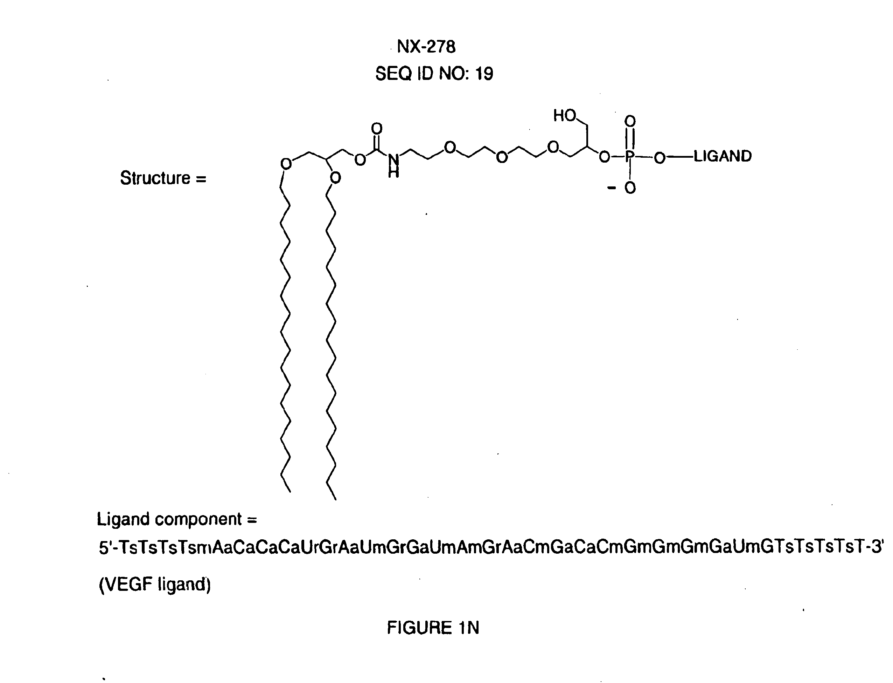

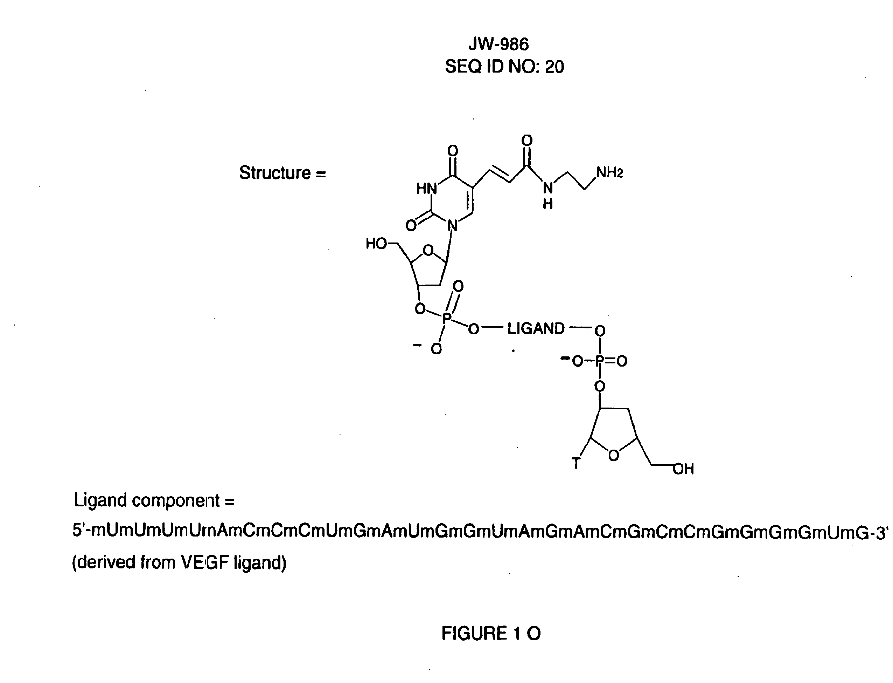

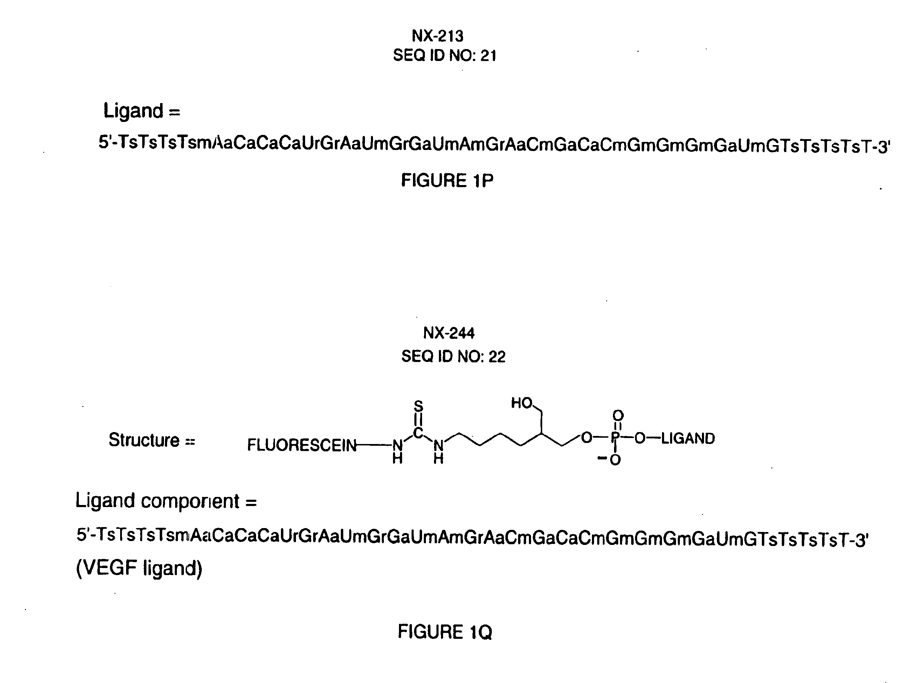

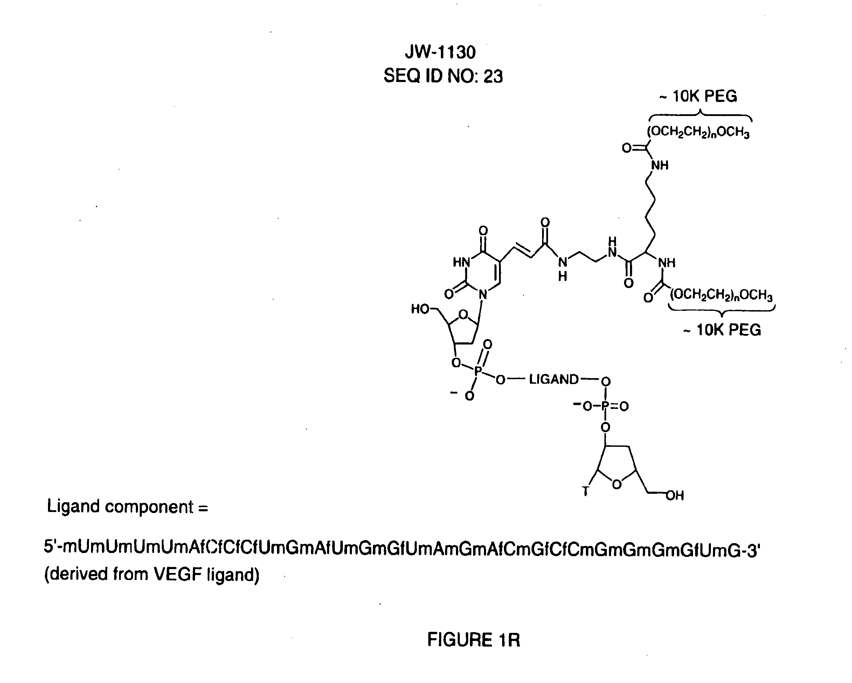

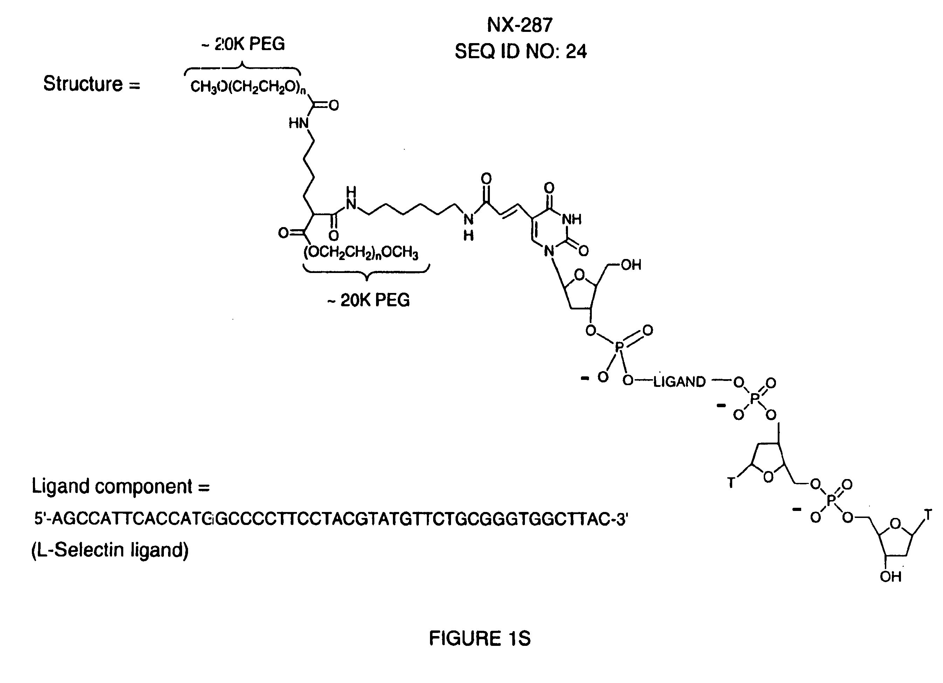

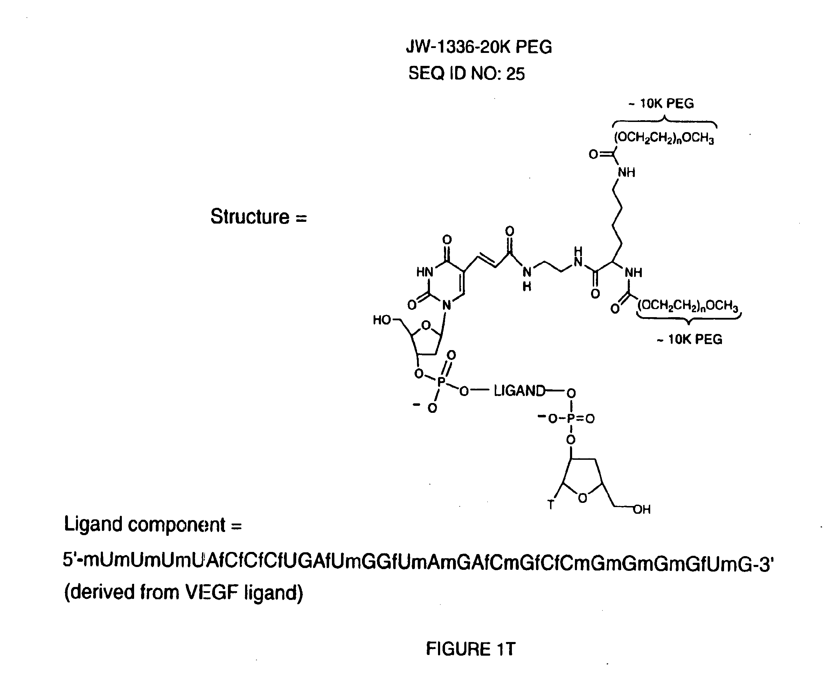

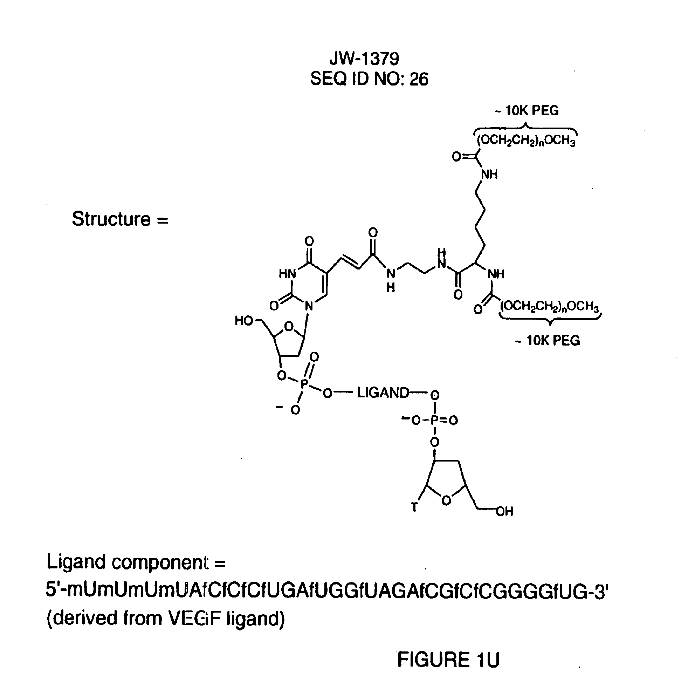

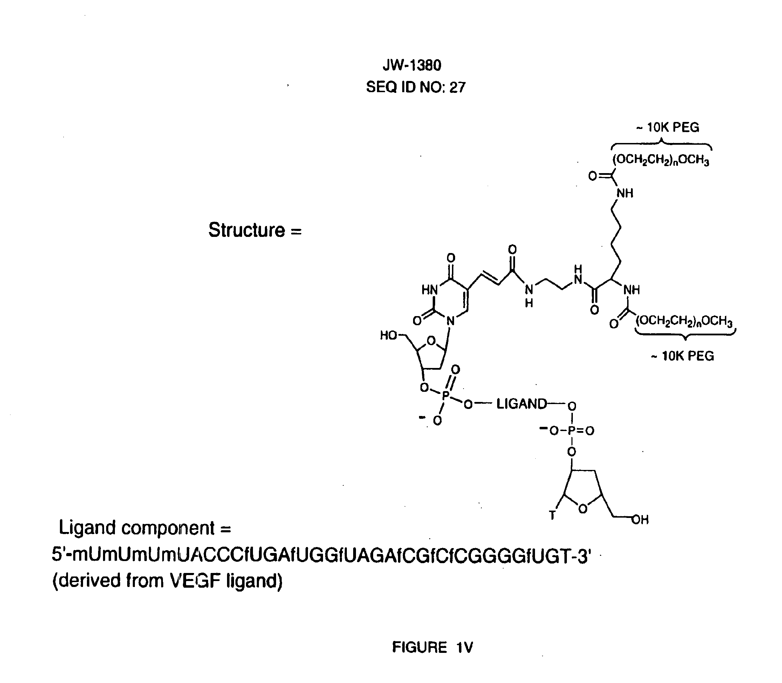

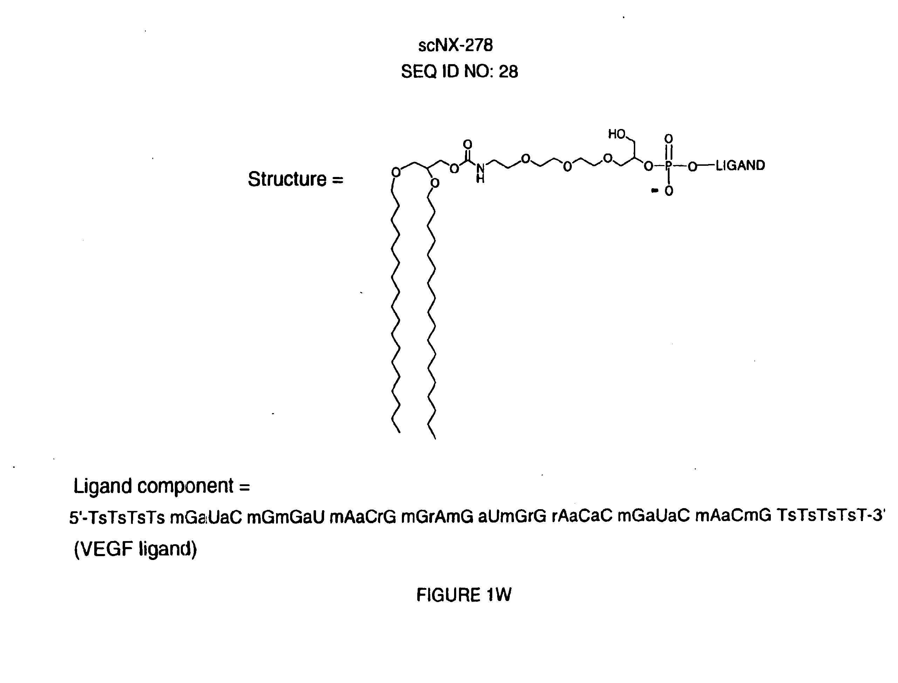

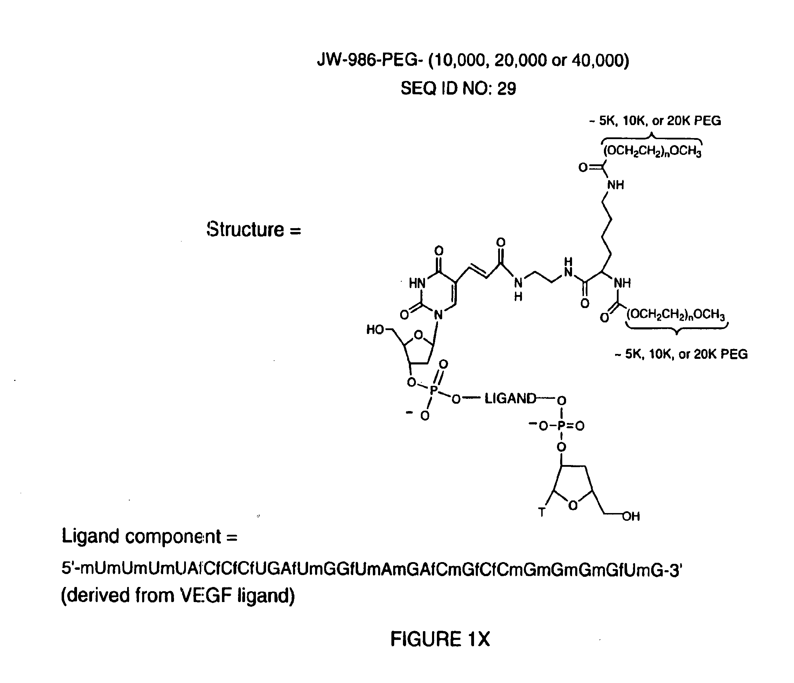

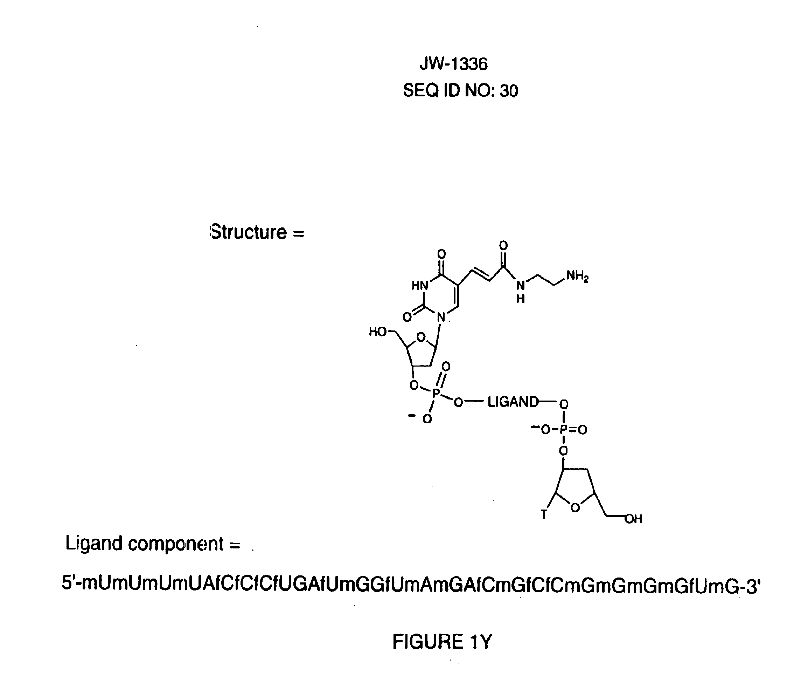

The following examples are provided to explain and illustrate the present invention and are not to be taken as limiting of the invention. The structures of the Nucleic Acid Ligands described in the examples below are shown in Figure 1. Example 1 describes the conjugation of Nucleic Acid Ligands with lipid, dialkyl glycerol or diacyl glycerol, as well as incorporation of pharmacokinetic modifiers via automated synthesis. Example 2 describes the conjugation of PEG and cholesterol with a Nucleic Acid Ligand. The modifications to the Nucleic Acid Ligand do not interfere with its ability to bind to its SELEX Target, as the binding affinities of the PEG-conjugated and cholesterylated Nucleic Acid Ligands were identical to the non-conjugated and non-cholesterylated molecules. Example 3 describes the pharmacokinetic properties of Nucleic Acid Ligands in association with cholesterol alone, dialkyl glycerol alone, with PEG alone, with cholesterol and Liposome, with dialkyl glycerol and Liposomes, and with PEG and Liposome. Nucleic Acid Ligands that have been modified at the 2' sugar position of purines and pyrimidines are also included. Example 4 describes the covalent conjugation of Nucleic Acid Ligands to Liposomes.

### EXAMPLE 1. LIPID, PEG, DIALKYL GLYCEROL AND DIACYL GLYCEROL REAGENTS FOR OLIGONUCLEOTIDE MODIFICATION

In this example, conjugation of Nucleic Acid Ligands with lipid and/or PEG or diacyl glycerol or diakyl glycerol reagents is described, as well as incorporation of the pharmacokinetic modifiers via automated synthesis using either phosphoramidite or H-phosphonate coupling chemistry. In the schemes depicted below, a solid arrow represents steps that have been completed, whereas a dashed arrow represents steps that have not yet been completed. **Scheme 1** depicts the preparation of a dipalmitoyl phosphatidyl ethanolamine maleimide reagent for coupling to sulfhydryl-modified oligonucleotide substrates. This procedure is analogous to that reported by Cheronis *et al* (Cheronis, J. C. et al., J. Med. Chem. (1992) 35:1563-1572) for the preparation of bis- and tris-maleimide reagents from simple, aliphatic di- and triamine substrates. Treatment of the phospholipid with methoxycarbonyl maleimide resulted in formation of an uncharacterized intermediate which, upon incubation with sulfhydryl-modified oligonucleotide, resulted in complete conversion of the oligonucleotide to the dipalmitoyl phosphatidyl ethanolamine conjugate (*vida infra*). Similar reagents are also available commercially from Avanti Polar Lipids.

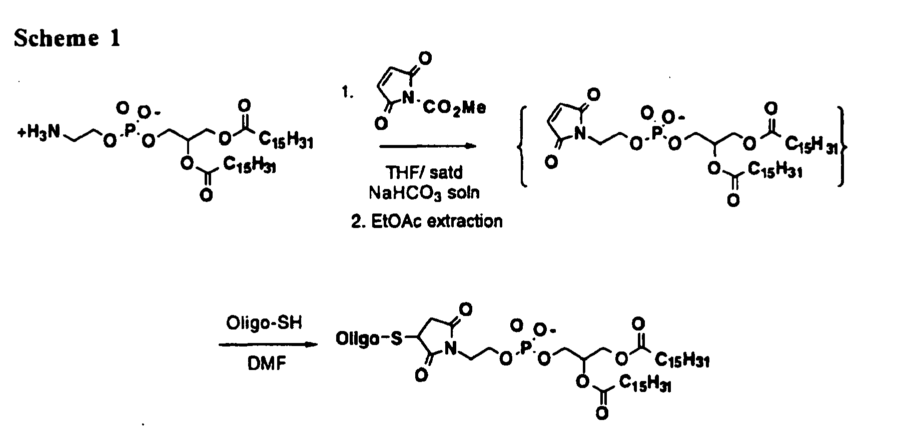

The ability to modify oligonucleotides under automated synthesis conditions has many obvious advantages. Reagents were prepared which allowed facile incorporation of lipid and/ or PEG moieties under standard automated synthesis conditions. Initially, the versatile module **10** was designed and synthesized (**Scheme 2**) which could be divergently converted to any number of oligo modification reagents of interest by simply chemoselectively functionalizing the amine group then phosphitylation of the hydroxyl group. Noteworthy is the flexibility this strategy offers with respect to the modification group (the amine ligand) and the activated phosphate precursor (phosphoramidite, H-phosphonate, or phosphate triester) introduced via derivatization of the 2'- hydroxyl group. Additionally, the glycerol nucleus of automated synthesis reagents prepared in this way renders the products suitable for oligo modification at internal chain positions, or at the 5' end. An alternative synthesis of module **10** is shown in **Scheme 3**. Tetraethylene glycol (1; TEG) is derivatized as the monotosylate **2a** upon treatment with a limiting amount of *p*-toluenesulfonyl chloride, preferably 10 mole percent, in a basic medium, preferably pyridine. In this manner, **2a** was obtained in 75% yield after silica gel filtration. Conversion of **2a** to the TEG phtalimide 3a was accomplished in 80% yield upon treatment with phthalimide in the presence of diazabicycloundecane (DBU) as a base at elevated temperature in DMF solution. Allylation of phthalimide **3a** (allyl bromide, NaH, THF/ DMF) afforded 65% yield of allyl TEG **4a**. Treatment of **4a** with 0.5% OsO₄ and 1.1 equivalents of N-methylmorpholine N-oxide (NMO) afforded a diol intermediate that was, without further purification, converted to the dimethoxytrityl (DMT) ether derivative **9** in 89% overall yield for the two steps. Finally, amine deprotection using 40% MeNH₂ was carried out to afford **10** in 95% purified yield. Module **10** has been further elaborated by treatment with PEG-nitrophenylcarbonate (PEG-NPC; Shearwater Polymers). In this way, the phosphitylation precursor **12** (**Scheme 4**) was prepared in excellent yield. Further conversions of **12** to both phosphoramidite **13** and H-phosphonate **14** have been carried out.

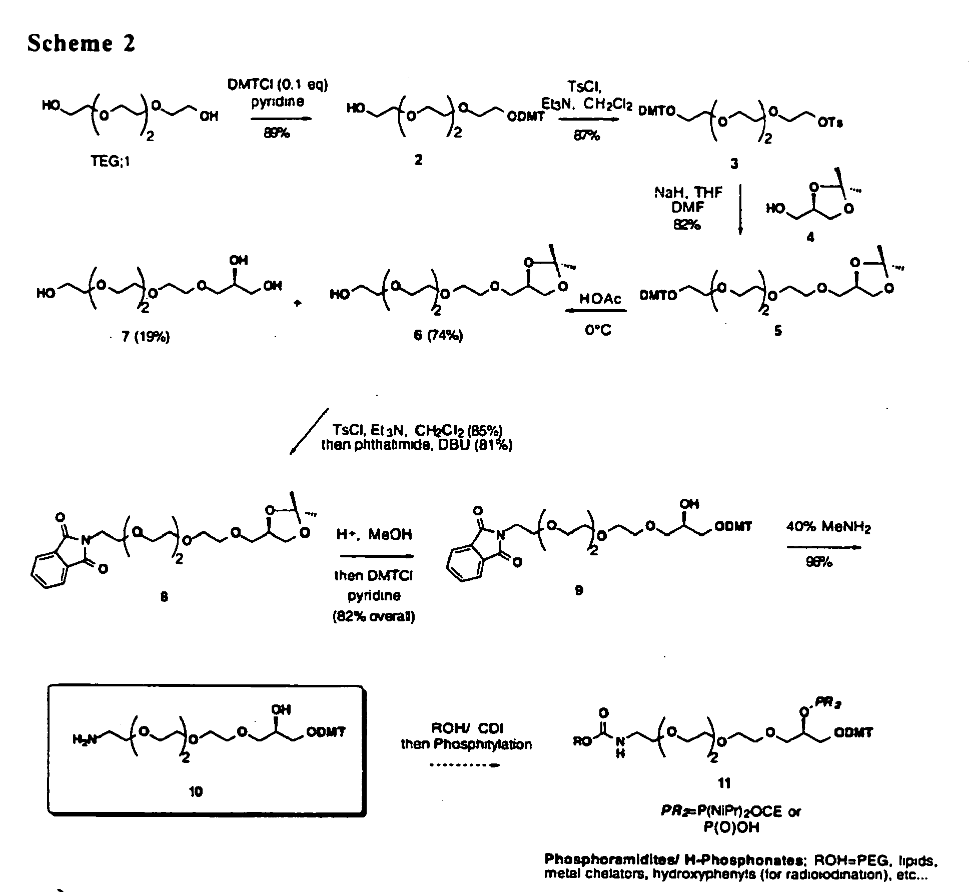

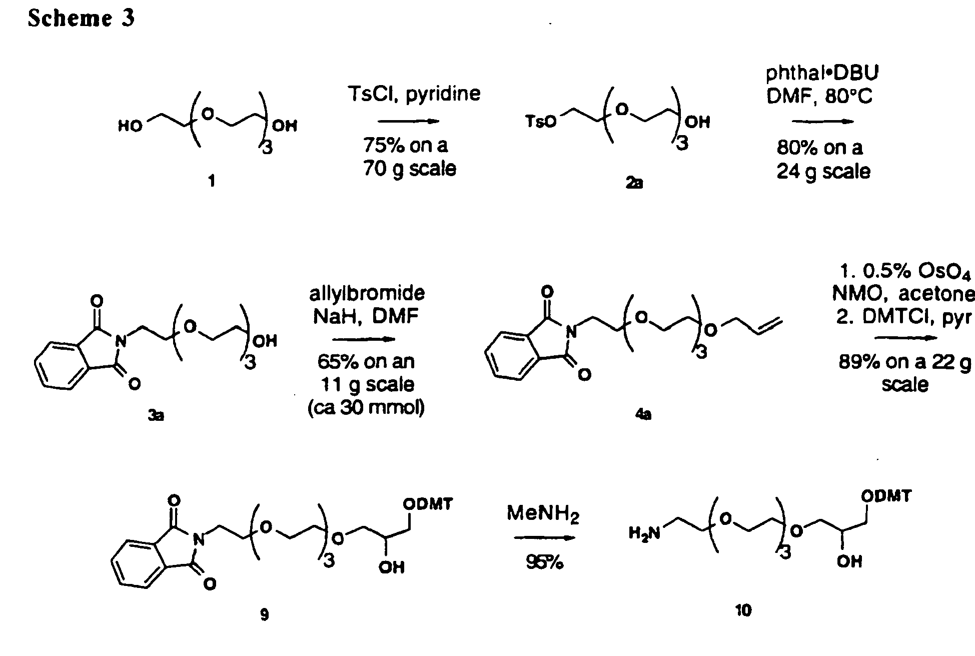

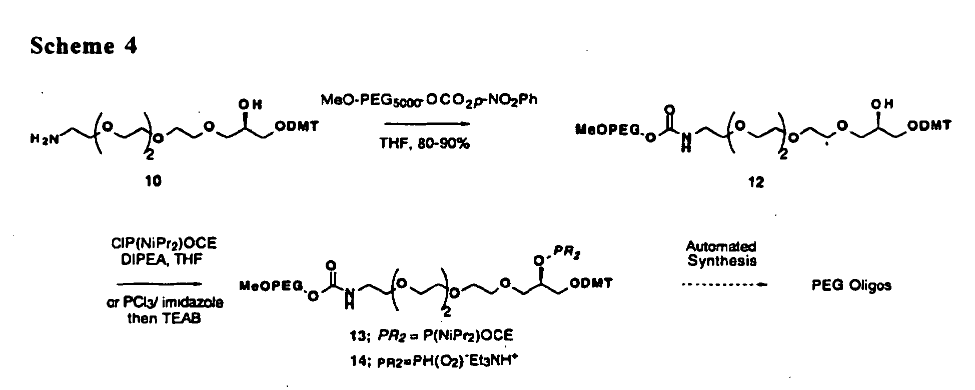

The design of a lipid reagent for oligonucleotide modification by automated synthesis necessitates replacement of the ester linkages of native diacyl glycerols, as in the dipalmitoyl phosphatidyl derivative described above, by glycerol-alkyl linkages stable to the basic deprotection protocol required for synthetic oligo recovery. The linkage that was chosen to explore initially was the ether linkage, as in the known dipalmityl glycerol derivative **15** (available from Sigma), although long-chain alkyl carbamates (or a combination of ethers and carbamates) would also be suitable. Dipalmityl glycerol was activated as the acyl carbonyl imidazole (CDI, pyridine) and this activated intermediate was coupled with the module **10** (pyridine, 80°C; 44%). Phosphitylation (ClP(iPr₂N)OCH₂CH₂CN; diisopropylethylamine (DIPEA), CH₂Cl₂; 59%) of **16** afforded the phosphoramidite **17** (**Scheme 5**). Synthesis of a C₁₈ analog of amidite 17 via a chloroformate intermediate is shown in **Scheme 6**. The dialkyl glycerol (**18**; DAG) was converted to the corresponding chloroformate **19** upon treatment with excess phosgene in toluene. Conjugation of **19** and amino alcohol **10** was carried out in pyridine to afford adduct **20** in 57% purified yield. Phosphitylation of the secondary hydroxyl of **20** under standard condition afforded phosphoramidite **21** in 95% yield. Coupling of amidite **17** to the 5'-end of a trinucleotide (TTT) on an ABI 394 automated oligonucleotide synthesizer using a slightly modified synthesis cycle with extended coupling times (2x 30 min couplings) for the lipid amidite resulted in 94+% coupling efficiency, as determined by in-line trityl cation analysis.

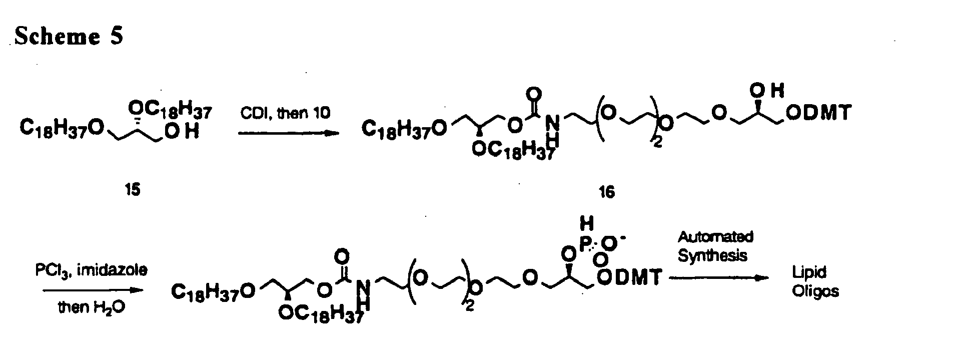

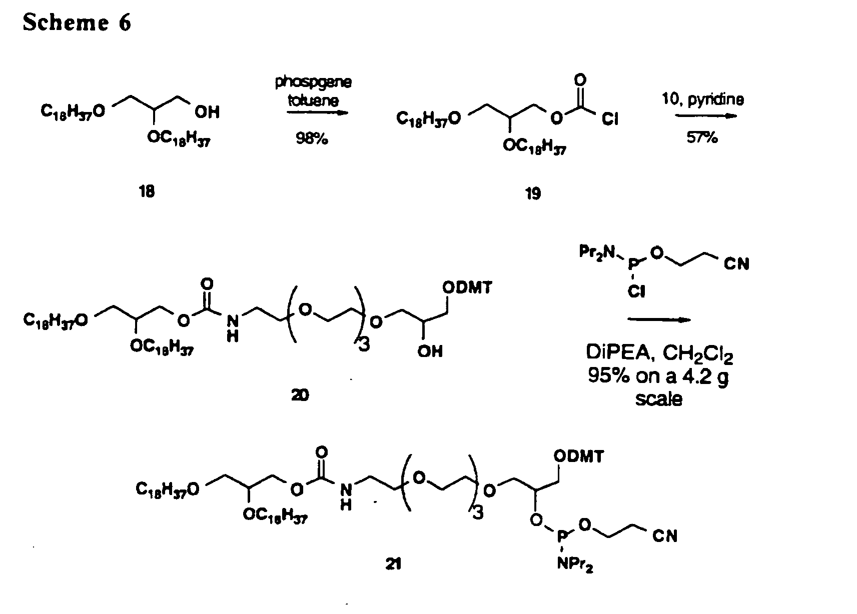

### Example A- Synthesis of Dipalmitoyl Phosphatidylethanolamine (DPPE)

Maleimide Reagent: A suspension of 200 mg (0.289 mmol) of DPPE and 54 mg (0.347 mmol) of methoxycarbonyl maleimide in 10 mL of THF/ saturated NaHCO₃ solution (1:1) was stirred at ambient temperature. After 12 h, the mixture was treated with 100 mL of EtOAc and the organic phase (which contained a gelatinous suspension of the product) separated from the aqueous phase. The organic phase was concentrated *in vacuo,* coevaporated twice with MeOH, and the resultant white solid triturated three times with EtOAc. This material was used without further characterization or purification in oligonucleotide conjugation experiments (*vida infra*).

### Example B: Synthesis and elaborations of automated Synthesis Module 10.

**Tetraethylene glycol dimethoxytrityl ether (2**): Tetraethylene glycol (76.4 mL, 0.44 mol) was dissolved in 300 mL of anhydrous pyridine and cooled to 0° C. 4,4'-dimethoxytrityl chloride (15 g, 0.044 mol) was added as a solid with stirring. The reaction flask was covered with a drying tube and the reaction was allowed to warm to ambient temperature overnight. The reaction was concentrated *in vacuo* at low temperature (<30° C). The residue was diluted with 300 mL of ethyl acetate and extracted with 3 x 300 mL of water. The combined aqueous layers were extracted with ethyl acetate, and the combined organic layers were dried over sodium sulfate and concentrated. The crude residue was purified by flash chromatography using 1000 mL of silica gel (wet-packed onto column with hexane containing 5% triethylamine), eluting with 10-20-40-60-80% ethyl acetate in hexane containing 5% triethylamine, and then ethyl acetate containing 5% triethylamine. 19.51 g (89%) of 2 was collected as a gold oil. ¹H NMR (300 MHz, CDCl3) d 7.47-7.16 (overlapping signals, 9H), 6.79 (d, 4H), 3.72 (s, 6H), 3.66-3.62 (m, 2H), 3.22 (t, J=5.22 Hz, 1H), 2.96 (br t, 1H); ¹³C NMR (75 MHz, CDCl₃) d 158.12, 144.86, 136.04, 129.81, 127.93, 127.49, 126.40, 112.78, 85.67, 72.31, 70.48, 70.44, 70.12, 62.89, 61.39, 54.89; Low resolution MS m/e calculated for C₁₅H₂₅O₇S (M-DMT+1⁺): 349.167, found 349.1.

**Tetraethylene glycol dimethoxytrityl ether *p*-toluenesulfonate (3):** Compound 2 (5.0 g, 10.06 mmol) was dissolved in 50 mL of anhydrous dichloromethane and cooled to 0° C. Triethylamine (1.82 mL, 13.1 mmol) was added, followed by p-toluenesulfonyl chloride (1.92 g, 10.06 mmol) as a solid, with stirring. The reaction was stored in the refrigerator overnight. TLC Analysis indicated the reaction was approximately 80% complete. An additional 0.5 equivalents oftriethylamine and 0.5 equivalents of p-toluenesulfonyl chloride were added, and the reaction was stirred at room temperature overnight. The reaction was filtered through Celite and concentrated. The residue was purified by flash chromatography using 300 mL of silica gel (wet-packed onto column using 5% triethylamine in hexane) eluting with 25-50-75% ethyl acetate in hexane containing 5% triethylamine, and then ethyl acetate containing 5% triethylamine. 5.7 g (87%) of 3 was collected as an orange oil. ¹H NMR (300 MHz, CDCl₃) d 7.75 (d, 2H), 7.44-7.12 (m, 11H), 6.78 (d, 4H), 4.12-4.09 (m, 2H), 3.73 (s, 6H), 3.66-3.54 (m, 13H), 3.22 (t, J=3.87 Hz, 2H), 2.41 (s, 3H).

**Tetraethylene glycol monotosylate (2a)**: Tetraethylene glycol (200 mL, 1.15 mol) was dissolved in 500 mL of pyridine and cooled to 0° C and treated with 22.0 g (0.115 mol) of p-toluenesulfonyl chloride. When solution was complete, the reaction mixture was stored in the refrigerator overnight, and then concentrated *in vacuo.* The residue was dissolved in 800 mL of EtOAc and extracted with 3 x 600 mL of H₂O. The H₂O fractions were back-extracted with EtOAc, and the combined EtOAc fractions were extracted with saturated aqueous Na₂HPO_{4.} The organic phase was dried over MgSO₄ and concentrated to a colorless oil The oil was purified by flash chromatography using 800 mL of silica gel and eluting with hexane, 25% EtOAc-50% EtOAc in hexane, then EtOAc, then 10% MeOH-20% MeOH in EtOAc to afford 23.7 g (60%) of pure product and 11% of product containing a minor impurity. 2a: ¹H NMR (300 MHz, CDCl₃) d 7.77 (d, J=8.1 Hz, 2H), 7.32 (d, J=8.1 Hz, 2H), 4.13 (t, J=4.8 Hz, 2H), 3.68-3.53 (m, 14H), 2.58 (t, J=5.6 Hz, 1H), 2.42 (s, 3H); ¹³C NMR (75 MHz, CDCl₃) d 168.2, 158.3, 144.8, 135.9, 133.8, 132.0, 129.9, 128.0, 127.7, 126.6, 123.1, 113.0, 85.9, 73.0, 70.6, 70.4, 70.0, 69.7,67.8,64.4,55.1,37.1; Low resolution MS m/e calculated for C₁₅H₂₄O₈S (M+1) : 349.1.

**Tetraethylene glycol monophthalimide (3a)**: To a stirred solution of 31.96 g (0.092 mol) of 2a in 400 mL of anhydrous DMF was added 14.2 g (1.05 equiv.) of phthalimide and 14.4 mL (1.05 equiv.) of 1,8-diazabicyclo[5.4.0]undec-7-ene. The solution was heated at 70° C for 18 h then concentrated *in vacuo.* The crude yellow oil was purified by flash chromatography using 1600 mL of silica gel and eluting with 25% EtOAc-50% EtOAc-75% EtOAc in hexane, then EtOAc, then 10% MeOH-20% MeOH in EtOAc to afford 23.8 g (80%) of **3a** as an oil. Upon standing, **3a** became a waxy white solid. ¹H NMR (300 MHz, CDCl₃) d 7.84-7.78 (m, 2H), 7.70-7.66 (m, 2H), 3.86 (t, J=5.6 Hz, 2H), 3.70 (t, J=5.6 Hz, 2H), 3.64-3.51 (m, 12H), 2.67 (bs, 1H); ¹³C NMR (75 MHz, CDCl₃) d 168.2, 133.8, 132.0, 123.1, 72.4, 70.5, 70.4, 70.2, 70.0, 67.8, 61.6, 37.2.

**Synthesis of compound 4a**: A solution of 15 g (0.0464 mol) of **3a** in 150 mL of THF and 15 mL of DMF was cooled to 0° C under Ar. Allyl bromide (6.0 mL, 1.5 equiv.) was added to the solution, followed by addition of 1.76 g (1.5 equiv.) of NaH as a solid. The opaque yellow suspension was stirred at 0° C for 30 minutes and then at room temperature for 18 hr. MeOH (50-100 mL) was added and concentrated then mixture was concentrated *in vacuo.* The crude material was purified by flash chromatography using 1500 mL of silica gel and eluting with 25% EtOAc-50% EtOAc-75% EtOAc in hexane, then EtOAc, then 10% MeOH in EtOAc to afford 11.05 g (65%) of **4a** as a yellow oil. ¹H NMR (300 MHz, CDCl₃) d 7.84-7.80 (m, 2H), 7.72-7.67 (m, 2H), 5.94-5.84 (m, 1H), 5.28-5.14 (m, 2H), 3.99 (d, J=5.61 Hz, 2H), 3.88 (t, J=5.85 Hz, 2H), 3.72 (t, J=5.76 Hz, 2H), 3.64-3.54 (m, 13H); ¹³C NMR (75 MHz. CDCl₃) d 168.0, 134.6, 133.7, 131.9, 123.0, 116.9, 72.0, 70.4, 69.9, 69.2, 67.7, 37.0.

**(S)-(+)-2,2-Dimethyl-1,3-dioxolanyl-4-ylmethyl (dimethoxytrityl)tetraethylene glycol (5):** Sodium hydride (0.56 g, 23.5 mmol) is weighed into a flame-dried flask, and 70 mL of anhydrous tetrahydrofuran and 15 mL of anhydrous N,N-dimethylformamide were added. The suspension was cooled to 0° C under argon, and (S)-(+)-2,2-dimethyl-1,3-dioxolane-4-methanol (2.7 mL, 21.7 mmol) was added dropwise via syringe. After stirring for 30 min at 0° C, compound **3** (11.77 g, 18.1 mmol) in 15 mL of tetrahydrofuran was added dropwise via addition funnel. The reaction mixture was stirred at ambient temperature overnight, then quenched with 100 mL of saturated aqueous sodium bicarbonate and diluted with 300 mL of diethyl ether. The layers were separated, and the ether layer was extracted 3 times with 300 mL of water. The ether layer was dried over sodium sulfate and concentrated. The residue was purified by flash chromatography using 500 mL of silica gel and eluting first with hexane and then 10-20-30-40-50-75% ethyl acetate in hexane and then with ethyl acetate. 8.93 g (82 %) of 5 was collected as a colorless oil. ¹H NMR (CDCl₃) d 7.46-7.43 (m, 2H), 7.34-7.17 (m, 7H), 6.78 (d, 4H), 4.23 (pentet, J=6.1 Hz, 1H), 4.00 (t, 8.2H), 3.75 (s, 6H), 3.71-3.60 (m, 15H), 3.53 (dd, J=10.0, 5.7 Hz, 1H), 3.52 (10.4, J=5.2 Hz, 1H), 1.39 (s, 3H), 1.33 (s, 3H); ¹³C NMR (75 MHz, CDCl₃) d 158.24, 144.97, 136.20, 129.93, 128.07, 127.61, 126.51, 112.89, 109.21, 85.77, 74.57, 72.20, 70.82, 70.59, 70.40, 69.68, 66.67, 63.01, 55.04, 26.67, 25.29; Low resolution MS m/e calculated for C₃₅H₅₀O₉N (M+NH₄⁺): 628.399, found 628.5.

**(S)-(+)-2,2-Dimethyl-1,3-dioxolanyl-4-ylmethyl tetraethylene glycol (6):** 100 mL of 80% acetic acid was cooled to 0° C and then added to compound **5** (6.6 g, 10.8 mmol) The clear orange solution was stirred at 0° C for 1 hr. Methanol (100 mL) was added, and the reaction mixture was concentrated *in vacuo* at low temperature (<30° C). The residue was purified by flash chromatography using 200 mL of silica gel, eluting first with ethyl acetate, and then 5-10-15-20% methanol in ethyl acetate. 2.5 g (74%) of **6** was collected as a colorless oil. ¹H NMR (CDCl₃) d 4.21 (pentet, J=6.2 Hz, 1H), 4.08 (dd, J=5.9, 4.8 Hz, 1H), 3.82-3.35 (m, 19H), 2.93 (br s, 1H), 1.34 (s, 3H), 1.28 (s, 3H); ¹³C NMR (75 MHz. CDCl₃) d 109.20, 74.50, 72.42, 72.14, 70.76, 70.39, 70.32, 70.14, 66.63, 61.48, 26.61, 25.23; Low resolution MS m/e calculated for C₃₅H₅₀O₉N (M+NH₄+): 628.399, found 628.5.

**(S)-(+)-2,2-Dimethyl-1,3-dioxolanyl-4-ylmethyl (phthalimido)tetraethylene glycol (8):** Alcohol 6 (4.06 g, 13.2 mmol) was dissolved in 50 mL of anhydrous dichloromethane and cooled to 0° C. Triethylamine (3.7 mL, 26.3 mmol) was added, followed by addition of p-toluenesulfonyl chloride (3.26 g, 17.1 mmol). The reaction flask was covered by a drying tube and allowed to warm to room temperature overnight. The reaction mixture was filtered through Celite, and the filtrate was concentrated *in vacuo.* The crude material was purified by flash chromatography on 400 mL of silica gel, eluting first with 10% ethyl acetate in hexane, and then 20-40-60-80-100% ethyl acetate, and then 10% methanol in ethyl acetate. Collected 5.21 g (85%) of the intermediate tosylate as a gold oil. (¹H NMR (400 MHz, CDCl₃) d 7.79 (d, J=8.1 Hz, 2H, tosyl aromatics), 7.32 (d, J=8.1 Hz, 2H, tosyl aromatics), 4.25 (pentet, J=6.0 Hz, 1H), 4.13 (t, 4.7H), 4.02 (dd, J=8.12, 6.4 Hz, 1H), 3.71-3.40 (m, 18H), 2.42 (s, 3H), 1.46 (s, 3H), 1.34 (s, 3H); ¹³C NMR (75 MHz, CDCl₃) d 144.74, 133.1, 129.76, 127.91, 109.8, 74.63, 72.27, 70.89, 70.68, 70.52, 70.44, 69.19, 68.60, 66.74, 64.1, 26.73, 25.34, 21.59; Low resolution MS m/e calculated for C₂₁H₃₈O₉NS (M+NH₄⁺): 480.364, found 480.2.) The tosylate (5.2 g, 11.24 mmol) was dissolved in 60 mL of anhydrous dimethylformamide. 1,8-Diazabicyclo-[5.4.0]undec -7-ene (1.7 mL, 11.24 mmol) was added, followed by phthalimide (1.65 g, 11.24 mol). The reaction mixture was heated to 70° C overnight. The reaction was concentrated *in vacuo,* and purified by flash chromatography on 400 mL of silica gel, eluting with 50% ethyl acetate in hexane. Collected 3.96 g (81 %) of **8** as a colorless oil. ¹H NMR (300 MHz, CDCl₃) d 7.83-7.79 (m, 2H), 7.72-7.68 (m, 2H), 4.26 (pentet, J=6.0 Hz, 1H), 4.03 (dd, J=8.2, 6.5 Hz, 1H), 3.88 (t, J=5.8 Hz, 1H), 3.74-3.44 (m, 18H), 1.39 (s, 3H), 1.33 (s, ³H); ¹³C NMR (75 MHz. CDCl₃) d 168.21, 133.88, 132.10, 123.19, 109.33, 74.66, 72.30, 70.90, 70.52, 70.04, 67.87, 66.79, 37.19, 26.75, 25.37; MS m/e calculated for C₂₂H₃₅O₈N₂ (M+NH₄⁺): 455.288, found 455.2.

**1-Dimethoxytriryl-3-(phthalimidotetraethylene glycolyl)-sn-glycerol (9):** According to **Scheme 2**, compound **9** is synthesized as follows: the acetyl **8** (5.16g, 11.8 mmol) was dissolved in 100 mL of anhydrous methanol, and anhydrous *p*-toluenesulfonic acid (100 mg) was added. The reaction flask was covered with a drying tube and the reaction was stirred at ambient temperature for 2.5 h, then neutralized by the addition of 10 mL of anhydrous pyridine, concentrated *in vacuo,* and coevaporated with anhydrous pyridine. The resulting diol was then dissolved in 1.50 mL of anhydrous pyridine and cooled to 0° C. 4,4 -Dimethoxytrityl chloride (4.39 g, 13 mmol) was added as a solid. The reaction flask was covered with a drying tube, and the reaction was allowed to warm to ambient temperature overnight. Methanol (50 mL) was added, and the reaction was concentrated *in vacuo.* The crude material was purified by flash chromatography on 700 mL of silica gel (wet-packed onto column with 5% triethylamine in hexane), eluting first with 10% ethylacetate in hexane (containing 5% triethylamine) and then 20-40-60-80-100% ethyl acetate (containing 5% triethylamine). Collected 6.98 g (82%) of **9** as a pale yellow oil. ¹H NMR (300 MHz, CDCl₃) d 7.80 (dd, J=5.4, 3.1 Hz, 2H), 7.68 (dd, J=5.4, 3.1 Hz, 2H), 7.42-7.14 (m, 9H, DMT), 6.79 (d, 4H, DMT), 3.95 (br m, 1H). 3.86 (t, J=5.9 Hz, 1H), 3.75 (s, 6H), 3.70 (t, J-5.6 Hz, 1H), 3.63-3.37 (m, 18H), 3.16 (m, 2H), 2.84 (br d, 1H); ¹³C NMR (75 MHz, CDCl₃) d 168.15, 158.32, 144.79, 135.95, 133.82, 132.02, 129.95, 128.04, 127.69, 126.64, 123.12, 112.97, 85.89, 72.97, 70.64, 70.43, 69.97, 69.74, 67.80, 64.34, 55.10, 37.14; Low resolution MS m/e calculated for C₄₀H₄₉O₁₀N₂ (M+NH₄⁺): 717.398, found 717.5. According to **Scheme 3**, compound **9** was synthesized as follows: To a stirred solution of **4a** (10.13 g, 0.0279 mol) in 100 mL of acetone and 1 mL of H₂O was added 3.98 g (1.22 equiv.) of N-methylmorpholine N-oxide. To this suspension was added 1.75 mL (0.005 equiv.) of Osmium tetroxide as a 2.5% solution in iPrOH. After addition of the OsO₄ solution, the reaction mixture became clear yellow. After TLC analysis indicated complete conversion of **4a** (ca 16 h), the reaction mixture was treated with 1.5 g of sodium hydrosulfite and 5.0 g of florisil and stirred 30 minutes. The suspension was filtered through florisil, the filtrate was concentrated to an oil. This crude product was combined with another batch prepared in the same manner from 1.0 g of **4a**. Two 100 mL portions of pyridine were co-evaporated from the combined lots and the residue was dissolved in 300 mL pyridine. The solution was cooled to 0° C and 10.89 g (1.05 equiv.) of 4,4'-dimethoxytrityl chloride was added. A drying tube was inserted in the flask and the reaction mixture was stirred at room temperature 16 h. The solution was treated with 20 mL of MeOH and concentrated *in vacuo,* keeping the temperature of the water bath below 40° C. The crude oil was purified by flash chromatography using 1100 mL of silica gel (wet-packed onto column using 3% triethylamine in hexane) and eluting with 10-100% EtOAc in hexane (all containing 3% triethylamine) to give 21.3 g (89% after two steps) of **9** as a yellow oil. ¹H NMR (300 MHz, CDCl₃) d 7.80-7.77 (m, 2H), 7.66-7.64 (m, 2H), 7.39-7.22 (m, 9H), 7.20-6.76 (m, 4H), 3.97 (bs, 1H), 3.84 (t, J=5.97 Hz, 2H), 3.74 (s, 6H). 3.68 (t, J=5.7 Hz, 2H), 3.60-3.49 (m, 14H), 3.13-2.76 (m, 2H), 2.00 (bs, 1H); ¹³C NMR (75 MHz, CDCl3) d 168.2, 158.3, 144.8, 135.9, 133.8, 132.0, 129.9, 128.0, 127.7, 126.6, 123.1, 113.0, 85.9, 73.0, 70.6, 70.4, 70.0, 69.7, 67.8, 64.4, 55.1, 37.1; Low resolution MS m/e calculated for C₄₀H₄₅O₁₀N (M+NH₄+) : 717.5.

**1-Dimethoxytrityl-3-(aminotetraethylene glycolyl)-*sn*-glycerol (10):** According to **Scheme 2**, compound **10** was synthesized as follows: Compound **9** (5.2 g, 7.2 mmol) was taken up in 50 mL of 40% methylamine in H₂O and 10 mL of methanol was added to solublize the starting material. The reaction mixture was heated at 50°C for 5 hr, and than was concentrated *in vacuo* and coevaporated with toluene. The crude material was purified by flash chromatography on 200 mL of silica gel, eluting with 15% methanolic ammonia in dichloromethane. Collected 3.94g (96%) of **10** as a pale yellow oil. ¹H NMR (300 MHz, CDCl₃) d 7.46-7.21 (m, 9H, DMT), 6.81 (d, 4H, DMT), 4.00 (m, 1H), 3.80 (s, 6H), 3.70-3.49 (overlapping m, 18H), 3.20 (dd, J=9.24, 5.49 Hz, 1H), 3.12 (dd, J=9.21, 6.0 Hz, 1H), 2.84-2.80 (m, 3H); ¹³C NMR (75 MHz, COCl₃) d 158.30, 144.82, 136.01, 129.95, 128.04, 127.66, 126.61, 112.95, 85.85, 73.46, 72.85, 70.55, 70.45, 69.99, 69.51, 64.43, 55.10, 41.40; Low resolution MS m/e calculated for C₃₂H₄₄O₈N (M+1⁺): 570.353, found 570.4.

According to **Scheme 3**, compound **10** was synthesized as follows: Compound **9** (5.2 g, 7.2 mmol) was taken up in 50 mL of 40% methylamine in H₂O and 10 mL of methanol was added to solublize the starting material. The reaction mixture was heated at 50°C for 5 hr, and than was concentrated *in vacuo* and coevaporated with toluene. The crude material was purified by flash chromatography on 200 mL of silica gel, eluting with 15% methanolic ammonia in dichloromethane. Collected 3.94g (96%) of **10** as a pale yellow oil. ¹H NMR (300 MHz. CDCl₃) d 7.46-7.21 (m, 9H, DMT), 6.81 (d, 4H. DMT), 4.00 (m, 1H), 3.80 (s, 6H), 3.70-3.49 (overlapping m, 18H), 3.20 (dd, J=9.24, 5.49 Hz, 1H), 3.12 (dd, J=9.21, 6.0 Hz, 1H), 2.84-2.80 (m, 3H); ¹³C NMR (75 MHz, CDCl₃) d 158.30, 144.82, 136.01, 129.95, 128.04, 127.66, 126.61, 112.95, 85.85, 73.46, 72.85, 70.55, 70.45, 69.99, 69.51,64.43, 55.10.41.40; Low resolution MS m/e calculated for C₃₂H₄₄O₈N (M+1⁺): 570.353, found 570.4.

**PEG Reagent 12:** To a stirred solution of 0.24 g (0.41 mmol) of **10** in 10 mL DMF was added 2.08 g (0.4 mmol) of methoxy-PEG₅₀₀₀-nitrophenyl carbonate (Shearwater Polymers, Inc.). The mixture was stirred 70 h then concentrated *in vacuo.* The residue was dissolved in EtOAc and the organic phase washed with three 30 mL portions of 10% NaOH solution. Purification by flash chromatography using 100 mL of silica gel (wet packed with dichloromethane containing 5% Et₃N) eluting with 5-10-15-20% methanolic ammonia in dichloromethane afforded 1.97 g (85%) of **12** as a white solid. ¹H NMR (300 MHz, CDCl₃) d 7.42-7.39 (d, 2H, DMT), 7.39-7.18 (m, 7H, DMT), 6.79 (d, 4H, DMT), 5.70 (br m, 1H), 4.21 (m, 1H), 3.97 (m, 1H), 3.88 (t, J=4.4 Hz, 1 H), 3.81 (s, 6H, DMT), 3.78-3.50 (br m, ∼500, PEG Hs), 3.42-3.31 (overlapping ms), 3.35 (s, PEG Me), 3.16 (m, 2H), 2.84 (br d, 4.2H); ¹³C NMR (75 MHz, CDCl₃) d 159.36, 157.19, 146.15, 136.95, 130.83, 128.86, 128.65, 127.61, 111.86,86.48, 73.58, 72.88, 72.5-70.0 (PEG carbons), 68.31, 65.77, 64.45, 41.36, 30.75 (unassigned impurity).

**PEG Phosphoramidite Reagent 13:** To a stirred solution of 2.22 g (0.4 mmol) of **12** in 60 mL of THF over 3 Å molecular sieves was added 0.24 mL (1.39 mmol) of diisopropylethylamine and 0.1 mL (0.44 mmol) of 2-cyanoethyl N,N-diisopropylchlorophosphoramidite. After 5 h, 3 1 P NMR indicated formation of desired product, as well as hydrolyzed phosphitylating agent and an additional 0.05 mL (0.22 mmol) of 2-cyanoethyl N,N-diisopropylchlorophosphoramidite was added. After 12 h, 0.07 mL (0.4 mmol) of DIPEA and 0.1 mL of the chlorophosphoramidite were added. The mixture was stirred 2 days, filtered through Celite and concentrated *in vacuo.* The residue was triturated with several portions of ether. ³¹P NMR d 156.4, 155.8. Also observed were signals at d 20.6, 19.8 presumed to correspond to hydrolyzed phosphitylation reagent.

**PEG H-Phosphonate Reagent 14:** To a stirred, 0°C solution of anhydrous imidazole (0.4 g; 5.9 mmol) in 10 mL of MeCN was added 0.17 mL (1.9 mmol) of PCl₃, followed by 0.86 mL (6.2 mmol) of Et₃N. To this mixture was added a solution of 3.0 g (0.55 mmol) of 12 in 12 mL of MeCN dropwise. The reaction mixture was allowed to warm to ambient temperature and stirred 16 h, treated with 10 mL of 0.1 M triethylammonium bicarbonate solution, and concentrated *in vacuo.* Triethylamine then toluene were coevaporated from the crude residue, then the product was dissolved in dichloromethane. The organic phase was washed with 1.0 M triethylammonium bicarbonate (TEAB) solution, dried over sodium sulfate, and concentrated. Purification by flash chromatography on 300 mL of silica gel (wet packed with hexane/ Et₃N (95:5)) eluting with 2-4-6-8-10-12-15% methanolic ammonia in dichloromethane afforded 1.95 g of product as a white solid. ³¹P NMR (CDCl₃) d 10.3, 10.2.

**Synthesis of Lipid phosphoramidite 17:** A solution of 540 mg (1 mmol) of 1,2-di-O-palmityl rac-glycerol in 10 mL of pyridine was treated with 195 mg (1.2 mmol) of carbonyldiimidazole and the resulting mixture stirred at ambient temperature overnight. To this mixture was added 570 mg (1 mmol) of the amino alcohol as a solution in 3 mL of DMF. The mixture was warmed to 40°C overnight, after which time ¹H NMR analysis of an aliquot from the reaction indicated very negligible formation of product. The mixture was heated to 80°C for 6 h (ca 1:1 product to starting material by ¹H NMR), then concentrated in vacuo. The crude residue was applied to a column of 125 mL of Si02 gel (Packed in hexanes) and the product eluted with a gradient of 20-50% EtOAc in hexanes (with 2% TEA) to afford 500 mg (44%) of intermediate **16** as a clear wax. (**16**: ¹H NMR (300 MHz, CDCl₃) d 7.42 (d, J=7.2 Hz, 2H), 7.30-7.18 (m, 7H), 6.76 (d, J=8.2 Hz, 4H), 5.34 (br t, 1H), 4.20-3.25 (overlapping signals), 3.16 (m, 2H), 1.53 (m), 1.24 (m), 0.86 (t, J=6.5 Hz, 6H). Alcohol **16** (500 mg; 0.44 mmol) was dissolved in 4 mL of CH₂Cl₂ and 0.15 mL (2 equiv) of DIPEA. To this solution was added 0.15 mL (0.66 mmol) of 2-cyanoethyl (N,N-diisopropylamino) chlorophosphoramidite. After 3 h, TLC showed conversion to 2 spots and the mixture was diluted with CH₂Cl₂ and washed with NaHCO₃ solution. The organic phase was dried over Na₂SO₄ and concentrated. The crude residue was applied to a column of 50 mL of SiO₂ gel (packed in hexanes) and the product eluted with 20 % EtOAc in hexanes (containing 2% TEA) to afford 350 mg (59%) of phosphoramidite **17** as a colorless wax. ³¹P NMR (CDCl₃) d 151.55, 151.08.

**Automated synthesis of lipid-oligo:** Phosphoramidite **17** was coupled to the 5'-end of a T-3mer (prepared by standard automated DNA synthesis on an ABI 394 instrument) using a modified coupling cycle consisting of two 30 minute exposures of an 0.1 M solution of **17** in 40% THF in MeCN to the column. In-line trityl analysis indicated a coupling efficiency of 94% for amidite **17**.

**Chloroformate 19:** To a stirred solution of 3 g (5.03 mmol) of 1,2-di-O-octadecyl-sn-glycerol **21** in 60 mL of toluene was added 20 mL of a 1.93 M solution of phosgene. Additional phosgene solution (2 X 10 mL; 15.4 equiv phosgene total) was added until no further alcohol starting material remained (by ¹H NMR analysis of concentrated aliquots). The excess phosgene and HCl was removed by aspirator and the reaction mixture was concentrated *in vacuo* to afford 3.3 g (98%) of the desired chloroformate **19** as a white powder. ¹H NMR (300 MHz, CDCl₃) d 4.45 (dd, J= 11.22, 3.69 Hz, 1H), 4.34 (dd, J=11.22, 6.15 Hz, 1H), 3.65 (m, 1H), 3.56-3.40 (m, 6H), 1.53 (m, 4H), 1.24 (m, 62H), 0.87 (t, J=6.36 Hz, 6H); ¹³C NMR (75 MHz, CDCl₃) d 75.90, 71.91, 71.35, 70.93, 69.36, 31.99, 29.96-29.44 (overlapping signals from hydrocarbon chains), 26.13, 26.04, 22.76, 14.18.

**Conjugate 20:** To a stirred solution of 2.25 g (3.95 mmol) of **10** in 60 mL of pyridine was added 2.6 g of the distearyl glycerol chloroformate **18**. ¹H NMR analysis of a concentrated aliquot after 2 h revealed no remaining chloroformate and the mixture was concentrated in vacuo. The crude residue was combined with material similarly prepared from 0.5 g (0.88 mmol) of **10** and 0.58 g of the chloroformate and the combined lots purified by flash silica gel chromatography on a column of 100 mL of silica gel (packed in hexanes containing 2% triethylamine) eluting with 200 mL hexanes, then 250 mL each of 10-20 and 30% EtOAc in hexanes, 500 mL 40% EtOAc in hexanes, then 250 mL each of 50-60-70 and 80% EtOAc in hexanes, and finally with 250 mL of EtOAc. The product containing fractions were concentrated to afford 3.3 g (57%) of the conjugate **20**.

**Phosphoramidite 21:** To a stirred solution or 3.8 g (3.26 mmol) of the conjugate in 25 mL of CH₂Cl₂ was added 1.14 mL (6.52 mmol) of diisopropylethylamine then 1.09 mL (4.88 mmol) of 2-cyanoethyl N,N-diisopropylchloro-phosphoramidite. After 2 hours, the mixture was diluted with CH₂Cl₂ and washed with saturated NaHCO₃ solution, dried over Na₂SO₄, and concentrated, The crude residue was purified by flash silica gel chromatography on a column of 125 mL of silica gel (packed in hexanes containing 2% triethylamine) eluting with 100 mL hexanes, then 250 mL each of 10 and 20% EtOAc in hexanes, 500 mL 30% EtOAc in hexanes, then 250 mL of 50% EtOAc in hexanes. The product containing fractions were concentrated to afford 4.2 g (95%) of the phosphoramidite 21. ³¹P NMR (CDCl₃) d 151.52, 151.08.

### EXAMPLE 2. PREPARATION AND FUNCTIONAL PROPERTIES OF PEG CONJUGATED AND CHOLESTEROL-DERIVATIZED NUCLEIC ACID LIGANDS.

### A PEG 3400 conjugate of a Nucleic Acid Ligand retains the binding affinity of the non-conjugated molecule

The ability of a bFGF ligand/PEG-3400 conjugate to bind bFGF was tested. Molecule 225t3 (SEQ ID NO:10), a high affinity DNA ligand to bFGF, was chosen for conjugation with PEG via a primary amine-NHS coupling reaction. Ligand 225t3 has a binding affinity of 1 nM and folds into a blunt ended hairpin with a Tm of 68°C. Ligand 225t3 was modified with a 3'-amino-modifier C7 CPG (Glen Research, Sterling, VA) using standard DNA synthesis methods and will be referred to as 225t3N (SEQ ID NO:11). 225t3N was reacted with the N-hydroxysuccinimidyl (NHS) active ester of PEG (avg. MW 3400) in 20% (v/v) dimethoxy formamide 80% (v/v) 0.5 M sodium bicarbonate buffered at pH 8.5. The resulting conjugate, 225t3N-PEG-3400 (SEQ ID NO: 14), was purified from the free DNA on a 12% polyacrylimide/7 M urea gel. The conjugate was 5' end-labeled with ³²P and a binding assay was performed. 225t3N-PEG-3400 (SEQ ID NO:14) binds to bFGF with the same affinity (K_{d}=1 nM) as 225t3.

### Conjugation of PEG-20.000 to a thrombin DNA ligand.

Thrombin DNA ligand NX256 (SEQ ID NO:9) (Figure 1D) containing an amino and a disulfide functionality was prepared using standard DNA synthesis methods and procedures on a Biosearch 8909 DNA/RNA synthesizer with dT-5'-LCAA-500 Å controlled-pore glass solid support and commercially available phosphoramidite reagents. Deprotection was followed by ion exchange HPLC purification. The 5' terminal disulfide bond was reduced by incubating the DNA in a solution 50 mM dithiolthreitol (DTT) at 37°C for 30 min.. The reduced DNA (containing a 5' terminal thiol) was run through a Nap-5 size exclusion column and the void volume, containing the DNA but not the DTT, was collected into a reaction vessel containing the maleimide derivatized PEG under an argon blanket. All solutions were purged with argon to remove oxygen. The reaction was kept at 40°C for 1 h. The progress of the reaction was monitored by removing small aliquots and analyzing them by electrophoresis on 8%/7 M urea polyacrylamide gels. At the end of the 1 hour incubation period, the reaction was essentially complete, and at this time an equal volume of methylene chloride was added to the reaction mix and the vessel shaken until a milky white suspension formed. The mix was spun at 14,000 rpm in an eppendorf centrifuge until the layers separated. The aqueous layer contained the free Nucleic Acid Ligand and was discarded. The product (PEG-20,000 modified DNA ligand NX256, referred to as NX256-PEG-20,000; SEQ ID NO:13) (Figure 1G) was further purified using ion exchange chromatography followed by reverse phase desalting and lyophilization to a white powder. This material was used to determine the effect of PEG modification on the pharmacokinetic behavior of the DNA Nucleic Acid Ligand (*vide infra*). PEG-10,000 modified ligand NX256, referred to as NX256-PEG-10,000, was prepared in an analogous manner.

### Tₘ Values for PEG conjugates

PEG functionality can also be introduced via the thiophosphate-maleimide reaction. We introduced the thiophosphate group into the thrombin DNA ligand at the 5'-end by the standard phosphoramidite method using commercially available reagents (this ligand is referred to as T-P4) (Figure 1F, SEQ ID NO: 12). The conjugation to the maleimide-containing PEG is analogous to the sulfhydryl-maleimide reaction described above. The PEG-conjugated ligands are referred to as T-P4-PEG-10,000 and T-P4-PEG-20,000 (SEQ ID NO:15). Melting temperatures (Tₘ) for T-P4-PEG-10,000, T-P4-PEG-20,000 and T-P4-DNA ligands were determined from the first derivative of the optical absorbance at 260 nm vs. temperature plots. The Tm values for T-P6-PEG-10,000, T-P6-PEG-20,000 and T-P6 were 40°C for all three ligands. These data and the bFGF-PEG-3400 Nucleic Acid Ligand binding data reported above, suggest that conjugation to PEG does not affect Nucleic Acid Ligand structure.

### A Cholesterylated bFGF ligand retains the binding affinity of the non-cholesterylated molecule

Cholesterol can be introduced into a Nucleic Acid Ligand, at any position in the sequence, by the standard solid phase phosphoramidite method. For example, we incorporated a tetraethyleneglycol cholesterol phosphoramidite (Glen Research, Sterling, VA) at the 3' end of ligand 225t3 (Figure 1E; SEQ ID NO:10) to produce ligand 225t3-Cholesterol Following purification on a 12% polyacrylimide/7 M urea gel, 225t3-Chol was 5' end-labeled with ³²P and a binding assay performed. The binding affinity of 225t3-Chol was identical (K_{d=}1 nM) to that of 225t3.

### EXAMPLE 3. PHARMACOKINETIC PROPERTIES OF CHOLESTEROL, DIACYL GLYCEROL, DIALKYL GLYCEROL, AND PEG MODIFIED DNAS.

The pharmacokinetic properties of thrombin DNA ligands NX229. NX232, NX253, NX253 + Liposome, and NX256-PEG20K were determined (see Figures 1A-1C, 1G for molecular descriptions) (SEQ ID NOS:6-8, 13). Each oligonucleotide was diluted in PBS to a solution concentration of 0.5-1.0 mg/ml based on UV absorption at 260 nm and an extinction coefficient of 0.033 µg oligo/ml. In all but one study, 6 rats received 0.5-1.0 mg oligonucleotide/kg animal weight and plasma samples were taken at various times from 1 minute to 4 hours. One rat was used in the study in which NX253 was tested. The plasma samples and quality control samples were analyzed using a hybridization assay. The hybridization assay utilized a capture oligonucleotide that contains a complementary sequence to the 5'-end of the DNA ligands conjugated to an iron oxide (FeO) bead (FeO-spacer-5'-d (GTC AGG CAC CAT CCC-3') (SEQ ID NO:1) where spacer = (dT)₈), and a detection oligonucleotide containing a biotin group at the 3'-end (5'd-CCC CAC TGA AGC ACC-spacer-3'-biotin-biotin, where spacer = (d1)₁₀) (SEQ ID NO:2). The amount of the biotin oligonucleotide attached to the bead was quantitated with the straptavidin-linked alkaline phosphatase, using CSPD-Sapphire as the luminescent substrate.

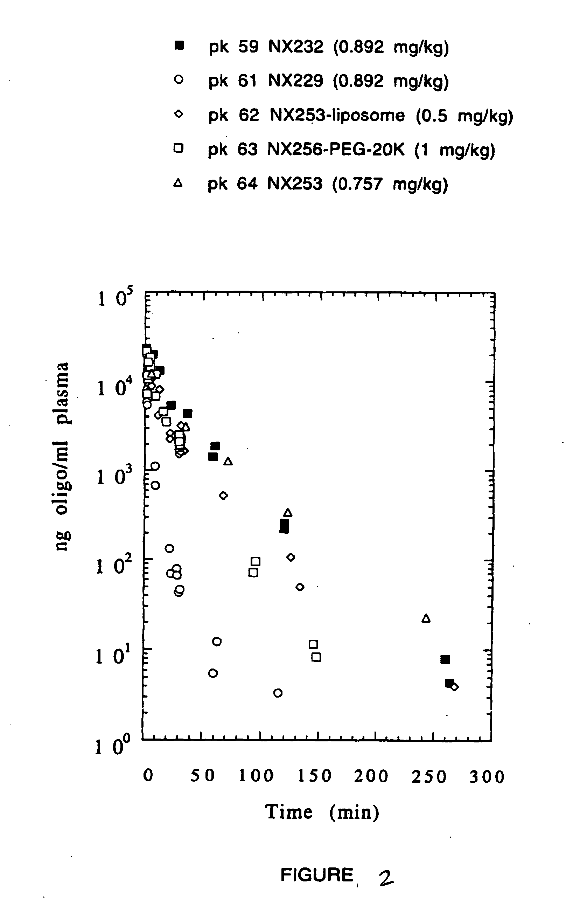

Data for the plasma concentration of NX229, NX232, NX253, NX253 + Liposome, and NX256-PEG20K (SEQ ID NOS:6-8,13) as a function of time following the bolus injection are summarized in Figure 2. The plasma concentrations of NX232, NX253, NX253 + Liposome, and NX256-PEG20K as a function of time are considerably greater compared to that of NX229 (SEQ ID NO:6). All of these oligonucleotides share the same thrombin binding module (d(CAG TCC GTG GTA GGG CAG GTT GGG GTG ACT TCG TGG)) (SEQ ID NO:3). The plasma concentration of an oligonucleotide as a function of time can be significantly increased by introducing appropriate functional groups into the oligonucleotide. Prolonged plasma half-life of a cholesterol-containing oligonucleotide compared to the control (non-cholesterol-containing) oligonucleotide has been observed previously (de Smidt et al., Nucl. Acids Res., 19: 4695 (1991))

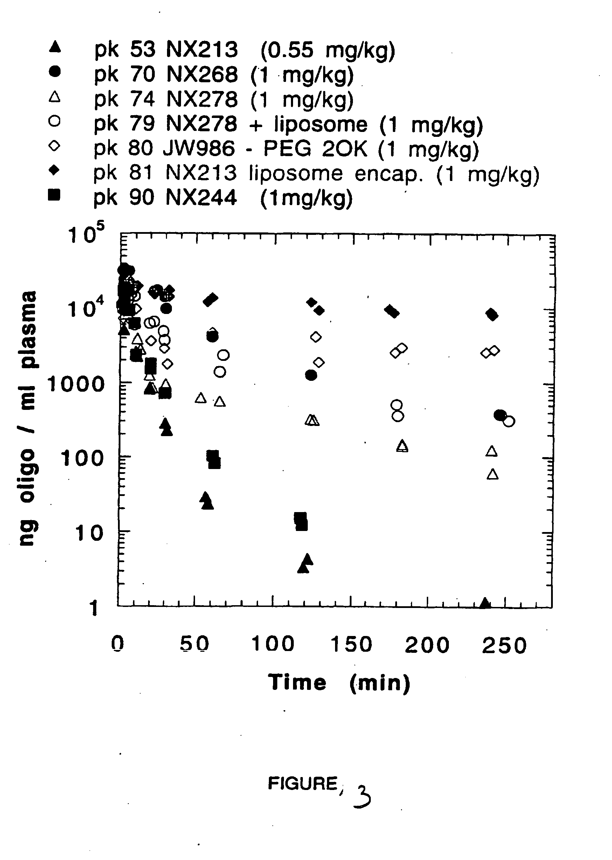

The plasma pharmacokinetic properties of a wide number of Nucleic Acid Ligands that have various functional groups attached to the base sequence of NX213 (see Figure 1 for molecular description), as well as some liposomal formulations of these oligonucleotides have been assessed. These data are summarized in Figure 3 (SEQ ID NOS:16, 19, 21, 22 and 29). The formulation with the slowest clearance rate was where NX213 (Figure 1P; SEQ ID NO:21) was encapsulated within liposomes.

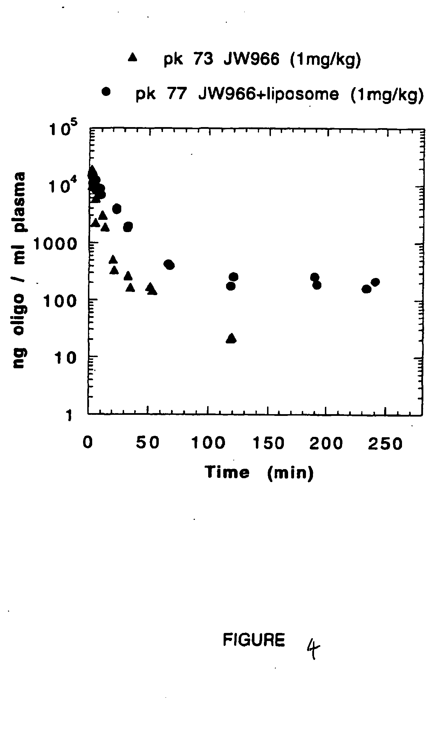

To determine the role of dialkyl glycerol DNA conjugates plus and minus liposomes PK studies 77 and 73 were carried out with the Thrombin DNA ligand dialkyl glycerol conjugate (See Figure 1 for molecular description). These data are summarized in Figure 4 (SEQ ID NO:18).

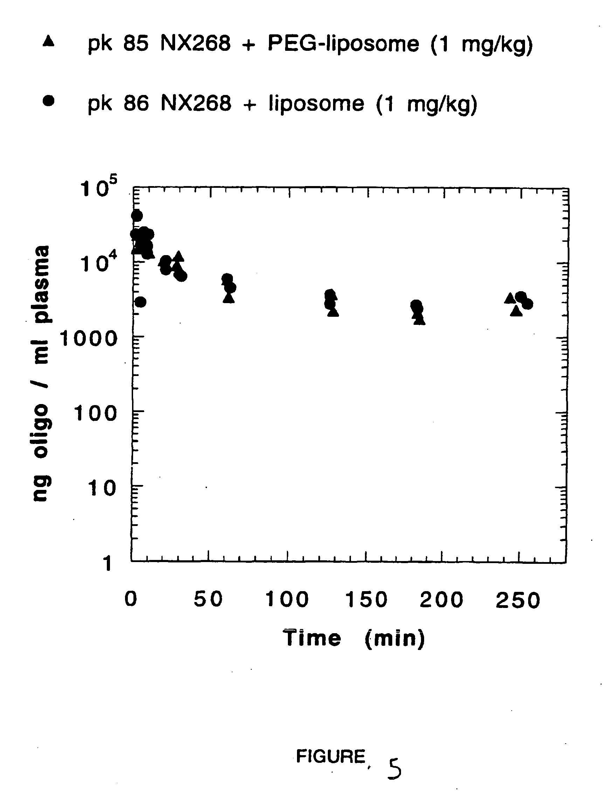

Cholesterylated VEGF oligonucleotides NX213 (NX268) (See Figure 1K for molecular description; SEQ. ID NO:16) were formulated with either PEG-liposomes or standard liposomes and the pharmacokinetics were evaluated (PK 85-86). These formulations contain oligonucleotides both on the inside and outside of the liposome. Figure 5 (SEQ ID NO:16) shows the rat plasma levels of full length oligonucleotides as a function of time after injection. Both liposome formulations show similar oligonucleotide pharmacokinetics.

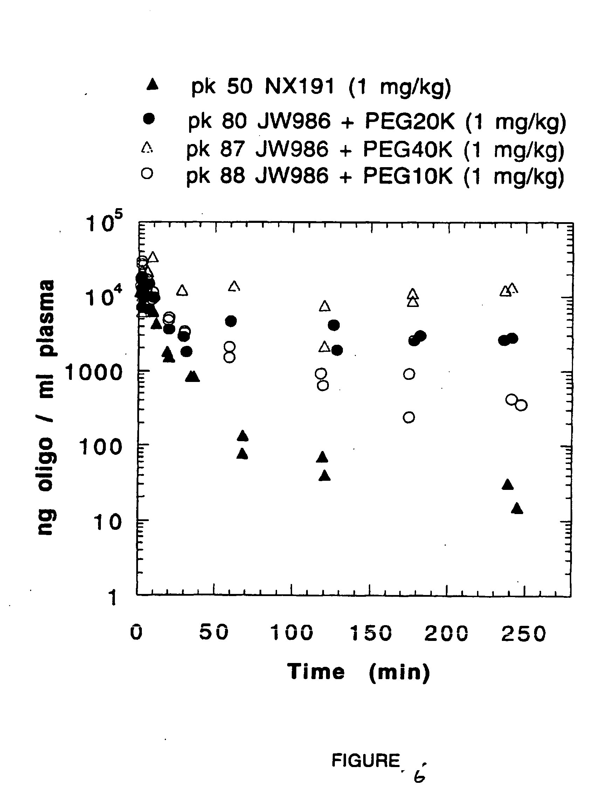

To evaluate size dependence on clearance, 2' O-methyl VEGF oligonucleotides with various PEG conjugates (See Figure 1 for molecular description) (PK 50,80,87, &88) have been studied. Figure 6 shows the comparison of the all 2' O-methyl oligonucleotides plus PEG 40K, 20K, 10K, as well as in the absence PEG (SEQ ID NOS:17 and 29). These data demonstrate significantly slower plasma clearance with increasing size of the PEG conjugate.

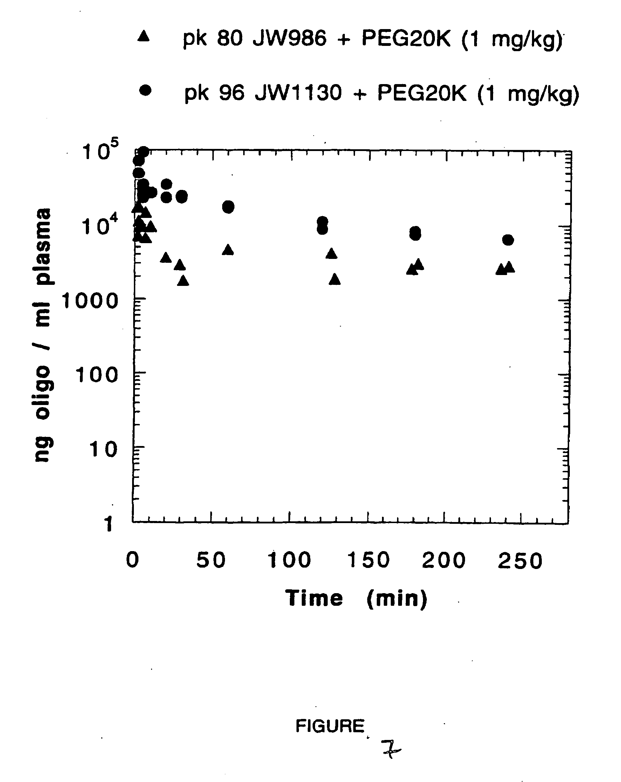

PK 96 was carried out to evaluate the pharmacokinetics of 2' F pyrimidines in conjunction with 2' O-methyl purines and PEG20K (JW1130) (See Figure 1R for molecular description; SEQ ID NO:23). Figure 7 shows the plasma levels of this oligonucleotide in comparison with the all 2' O-methyl version (PK 80, JW986) (See Figure 1X for molecular description; SEQ ID NO:29). These data show fairly similar clearance properties for both oligonucleotides.

The observation that JW1130 (SEQ ID NO:23), containing 2' F pyrimidines, shows similar clearance to JW986 (2' O-methyl pyrimidines) suggests that oligonucleotides with 2'F pyrimidines are resistant to nuclease digestion.

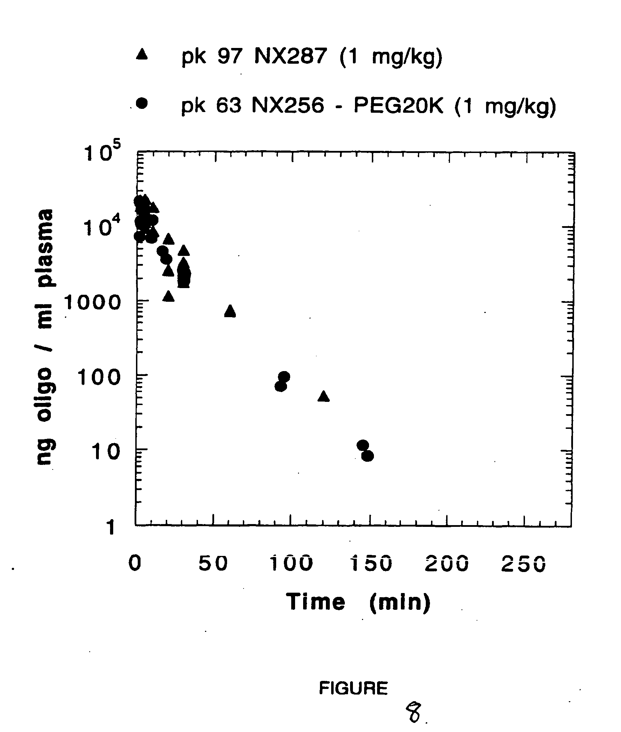

PK 97 was carried out to determine the clearance properties of a L-Selectin DNA ligand (NX287) (Figure 1S; SEQ ID NO:24) conjugated with 40K PEG. Figure 8 shows the plasma levels of this oligonucleotide as a function of time after bolus injection (dose 1 mg/kg). For comparison, Thrombin DNA ligand (NX256) (Figure 1H; SEQ ID NO:13) conjugated with 20K PEG was included. As shown in Figure 8 these two oligonucleotides show similar clearance rates presumably due to metabolism.

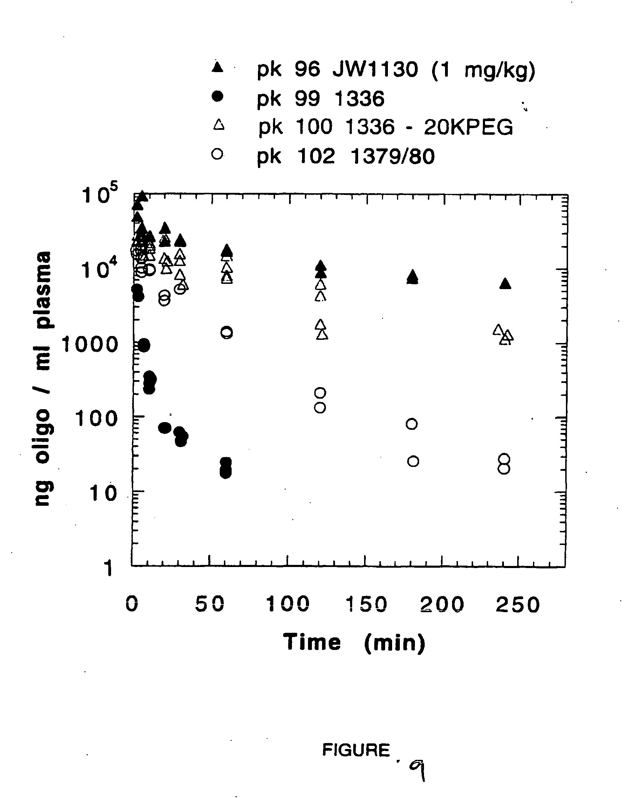

PK studies 99, 100 and 102 have been carried out as part of a larger study to assess the stability of oligonucleotides *in vivo*. These studies are shown in Figure 9. For comparison, PK 96 (JW1130 VEGF) 2'F Py 2'O-Met (14 Pu) + PEG 20K) (Figure 1R; SEQ ID NO:23) is also included. The oligonucleotides used in PK studies 96, 100, and 102 differ only in the number of purine positions that contain 2' deoxy nucleotides, where PK 96 contains no 2' deoxy purines, PK 100 four 2' deoxy purines, and in PK 102 all 14 purines are 2' deoxy. Figure 9 (SEQ ID NOS:23, 25, 26, 27 and 30) demonstrates a clear relationship between increasing clearance rate and the number of deoxy nucleotides present in the oligonucleotide. This observed increase in clearance rate with increasing number of deoxy nucleotides is assumed to be due to increased metabolism of these oligonucleotides. An encouraging observation is the high level of stability shown for PK 100 containing four 2' deoxy purines, and suggests that post-SELEX modification may be appropriate if a large number of purines can be modified. Also shown in Figure 9 is PK 99 vs. PK 100, that differ in PEG20K conjugation. As previously observed with other oligonucleotides, conjugation to PEG molecules of significant molecular weight dramatically reduces the observed clearance rate from plasma.

### EXAMPLE 4. COVALENT CONJUGATION OF NUCLEIC ACID LIGANDS TO LIPOSOMES

In scheme 1 provided below, a heterobifunctional PEG-2000 (PEG with molecular weight 2000 Da) containing a N-hydroxysuccinimide ester and vinyl sulfone functionalities was first conjugated to a Liposome containing 2 mole % distearylphosphatidylethaholamine (DSPE) via the N-hydroxysuccinimide ester moiety. The product was purified from the free PEG by size exclusion chromatography. The vinyl sulfone product was then allowed to react with reduced NX 256 (Figure 1D; SEQ ID NO: 9). A DSPE-PEG-2000-vinyl sulfone is commercially available and can be used to manufacture Liposomes that contain the vinyl sulfone functionality, thus eliminating a conjugation step from Scheme 1.

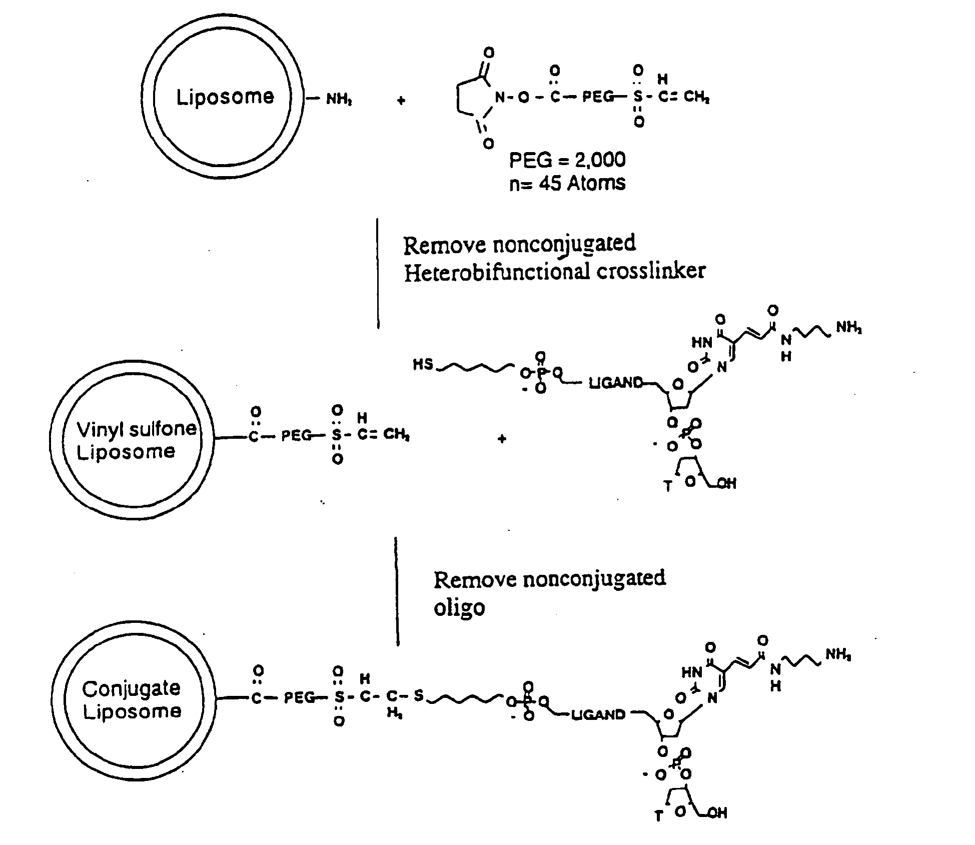

The second reaction, shown below as scheme 2, has been completed. The starting material was a distearylphosphatidylcholine (DSPC) Liposome containing 2 mole % DSPE maleimide. Using a value of 50,000 lipids per Liposome, the Liposome should have approximately 1000 maleimide molecules per Liposome, about 600 of which are available on the outside. The Nucleic Acid Ligand-Liposome Complex was separated from the free Nucleic Acid Ligand via size exclusion chromatography (*vide supra*). From the absorbance at 260 nm, it was estimated that approximately 200 molecules of the Nucleic Acid Ligand were conjugated to each Liposome.

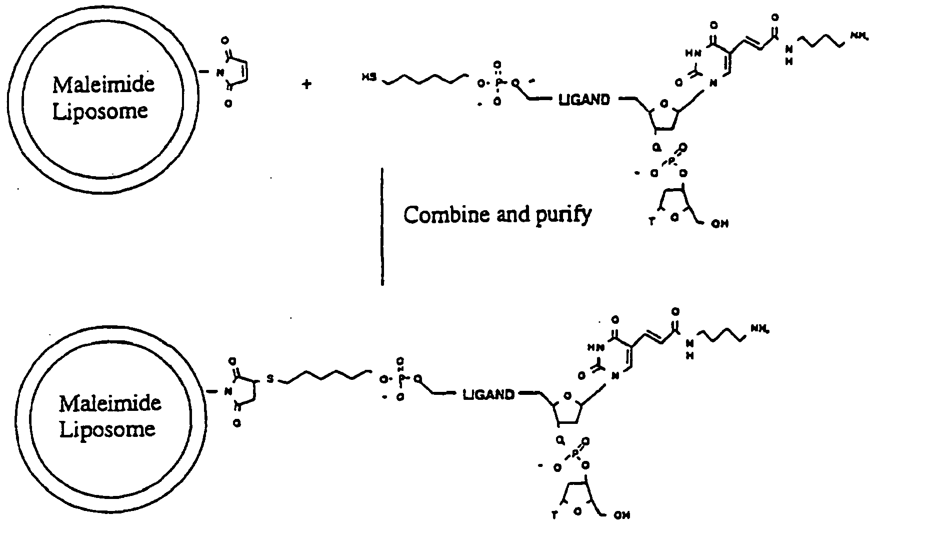

## Claims

1. A method for the preparation of a therapeutic or diagnostic complex comprising one single stranded nucleic acid ligand and a polyethylene glycol covalently bound at the 5' or 3' terminus of the nucleic acid ligand, wherein the nucleic acid ligand has high affinity and specificity for a target molecule other than a polynucleotide that binds to the Nucleic Acid Ligand through a mechanism which depends on Watson/Crick base pairing or triple helix binding and wherein the polyethylene glycol has a molecular weight of 1000Da or more, said method comprising covalently binding a single stranded nucleic acid ligand to polyethylene glycol having a molecular weight of 1000Da or more at the 5' or 3' terminus of the nucleic acid ligand, wherein the method comprises identifying the nucleic acid ligand from a candidate mixture of nucleic acids by a method comprising the steps:
(a) contacting a candidate mixture of single stranded nucleic acids with the target molecule under conditions favourable for binding;
(b) partitioning unbound nucleic acids from those nucleic acids that have bound to target molecules;
(c) dissociating the nucleic acid-target pairs;
(d) amplifying the nucleic acids dissociated from the nucleic acid-target pairs to yield a ligand-enriched mixture of nucleic acids;
wherein steps (a)-(d) are reiterated through as many cycles as desired to yield a nucleic acid ligand to the target molecule.

2. The method of claim 1 wherein the method comprises covalently binding polyethylene glycol to the 5' or 3' hydroxyl group of the nucleic acid ligand.

3. The method of claim 1 or 2 wherein the method comprises covalently binding polyethylene glycol to the 5' thiol through a maleimide or vinyl sulfone functionality.

4. The method of any one of claims 1 to 3 wherein the method comprises covalently binding a linker molecule to the 5' or 3' terminus of the nucleic acid ligand and covalently binding the linker molecule to the polyethylene glycol.

5. The method of any one of claims 1 to 4 wherein the nucleic acid ligand has a 3'3' inverted phosphodiester linkage at the 3' end.

6. The method of any one of claims 1 to 5 wherein the nucleic acid ligand is DNA.

7. The method of any one of claims 1 to 5 wherein the nucleic acid ligand is RNA.

8. The method of any one of claims 1 to 7 wherein the nucleic acid ligand is chemically modified.

9. The method of claim 8 wherein the modification is chosen from the group:
- 2'-position sugar modifications;
- 5-position pyrimidine modifications;
- 8-position purine modifications;
- modifications at exocyclic amines;
- substitution of 4-thiouridine;
- substitution of 5-bromo-uracil;
- substitution of 5-iodo-uracil;
- backbone modifications;
- phosphorothioate linkages;
- alkyl phosphate;
- methylations;
- 5' phosphorothioate capping;
- 3' phosphorothioate capping.

## Patentansprüche

1. Verfahren zur Herstellung eines therapeutischen oder diagnostischen Komplexes, der einen einzelsträngigen Nucleinsäureliganden und ein kovalent an den 5'-oder 3'-Terminus des Nucleinsäureliganden gebundenes Polyethylenglykol umfasst, wobei der Nucleinsäureligand hohe Affinität und Spezifität für ein anderes Target-Molekül als ein Polynucleotid aufweist, das durch einen auf Watson/Crick-Basenpaarung oder dem Tripelhelixbindung basierenden Mechanismus an den Nucleinsäureliganden bindet, und wobei das Polyethylenglykol ein Molekulargewicht von 1000 Da oder mehr aufweist, wobei das Verfahren das kovalente Binden eines einzelsträngigen Nucleinsäureliganden an ein Polyethylenglykol mit einem Molekulargewicht von 1000 Da oder mehr umfasst, und zwar am 5'- oder 3'-Terminus des Nucleinsäureliganden, wobei das Verfahren das Identifizieren des Nucleinsäureliganden aus einem Kandidatengemisch von Nucleinsäuren durch ein folgende Schritte umfassendes Verfahren umfasst:
(a) das Kontaktieren eines Kandidatengemischs von einzelsträngigen Nucleinsäuren mit dem Target-Molekül unter Bedingungen, die für eine Bindung günstig sind;
(b) das Trennen ungebundener Nucleinsäuren von jenen Nucleinsäuren, die an die Target-Moleküle gebunden haben;
(c) das Dissozüeren der Nucleinsäure-Target-Paare;
(d) das Amplifizieren der von den Nucleinsäure-Target-Paaren dissoziierten Nucleinsäuren, um ein mit Liganden angereichertes Gemisch von Nucleinsäuren zu erhalten;
wobei die Schritte (a)-(d) in so vielen Zyklen wie gewünscht wiederholt werden, um dem Target-Molekül einen Nucleinsäureliganden bereitzustellen.

2. Verfahren nach Anspruch 1, wobei das Verfahren das kovalente Binden von Polyethylenglykol an die 5'- oder 3'-Hydroxylgruppe des Nucleinsäureliganden umfasst.

3. Verfahren nach Anspruch 1 oder 2, wobei das Verfahren das kovalente Binden von Polyethylenglykol an das 5'-Thiol durch eine Maleinimid- oder Vinylsulfonfunktionalität umfasst.

4. Verfahren nach einem der Ansprüche 1 bis 3, wobei das Verfahren das kovalente Binden eines Linker-Moleküls an den 5'- oder 3'-Terminus des Nucleinsäureliganden und das kovalente Binden des Linker-Moleküls an das Polyethylenglykol umfasst.

5. Verfahren nach einem der Ansprüche 1 bis 4, wobei der Nucleinsäureligand eine 3'3'-invertierte Phosphodiesterbindung am 3'-Ende aufweist.

6. Verfahren nach einem der Ansprüche 1 bis 5, wobei der Nucleinsäureligand DNA ist.

7. Verfahren nach einem der Ansprüche 1 bis 5, wobei der Nucleinsäureligand RNA ist.

8. Verfahren nach einem der Ansprüche 1 bis 7, wobei der Nucleinsäureligand chemisch modifiziert ist.

9. Verfahren nach Anspruch 8, wobei die Modifikation aus folgender Gruppe ausgewählt ist:
- 2'-Position-Zuckermodifikationen;
- 5-Position-Pyrimidinmodifikationen;
- 8-Position-Purinmodifikationen;
- Modifikationen an exozyklischen Aminen;
- Substitution von 4-Thiouridin;
- Substitution von 5-Bromuracil;
- Substitution von 5-loduracil;
- Rückgratmodifikationen;
- Thiophosphatbindungen;
- Alkylphosphat;
- Methylierungen;
- 5'-Thiophosphat-Capping;
- 3'-Thiophosphat-Capping.

## Revendications

1. Méthode de préparation d'un complexe thérapeutique ou diagnostic comprenant un ligand d'acide nucléique à simple brin et un polyéthylène glycol lié de manière covalente au terminal 5' ou 3' du ligand d'acide nucléique, où le ligand d'acide nucléique a une affinité et spécificité élevées pour une molécule cible autre qu'un polynucléotide qui se lie au Ligand d'Acide Nucléique par un mécanisme qui dépend de l'appariement de bases Watson/Crick ou d'une liaison hélice triple, et où le polyéthylène glycol a un poids moléculaire de 1000 Da ou plus, ladite méthode comprenant la liaison covalente d'un ligand d'acide nucléique à simple brin au polyéthylène glycol ayant un poids moléculaire de 1000 Da ou plus au terminal 5' ou 3' du ligand d'acide nucléique, où la méthode comprend l'identification du ligand de l'acide nucléique à partir d'un mélange candidat d'acides nucléiques par une méthode comprenant les étapes:
(a) mettre en contact un mélange candidat d'acides nucléiques à simple brin avec la molécule cible sous des conditions favorables pour la liaison;
(b) séparer les acides nucléiques non liés des acides nucléiques qui se sont liés aux molécules cibles;
(c) dissocier les paires d'acides nucléiques-cibles;
(d) amplifier les acides nucléiques dissociés des paires d'acides nucléiques cibles pour obtenir un mélange enrichi en ligands des acides nucléiques;
où les étapes (a)-(d) sont répétées au nombre de cycles souhaités pour obtenir un ligand d'acide nucléique à la molécule cible.

2. Méthode selon la revendication 1, où la méthode comprend la liaison covalente du polyéthylène glycol au groupe hydroxyle 5' ou 3' du ligand de l'acide nucléique.

3. Méthode selon la revendication 1 ou 2, où la méthode comprend la liaison covalente du polyéthylène glycol au thiol 5' par une fonctionnalité de maléimide ou vinyle sulfone.

4. Méthode selon l'une quelconque des revendications 1 à 3, où la méthode comprend la liaison covalente d'une molécule de liaison au terminal 5' ou 3' du ligand de l'acide nucléique et la liaison covalente de la molécule de liaison au polyéthylène glycol.

5. Méthode selon l'une quelconque des revendications 1 à 4, où le ligand de l'acide nucléique présente une liaison de phosphodiester inversée 3'3' à l'extrémité 3'.

6. Méthode selon l'une quelconque des revendications 1 à 5, où le ligand de l'acide nucléique est l'ADN.

7. Méthode selon l'une quelconque des revendications 1 à 5, où le ligand de l'acide nucléique est l'ARN.

8. Méthode selon l'une quelconque des revendications 1 à 7, dans laquelle le ligand de l'acide nucléique est chimiquement modifié.

9. Méthode selon la revendication 8, dans laquelle la modification est sélectionnée dans le groupe:
- modifications de sucre à la position 2';
- modifications de pyrimidine à la position 5;
- modifications de purine à la position 8;
- modifications et amines exocycliques;
- substitution de 4-thiouridine;
- substitution de 5-bromo-uracil;
- substitution de 5-iodo-uracil;
- modifications de l'ossature;
- liaisons de phosphorothioate;
- phosphate d'alkyle;
- méthylations;
- coiffage de phosphorothioate 5';
- coiffage de phosphorothioate 3'.
